# DEMANDE DE BREVET EUROPEEN

(11) **EP 3 517 136 A1**
(43) Date de publication de la demande: **31.07.2019**
(21) Numéro de dépôt: 19159482.9
(22) Date de dépôt: 02.05.2011
(51) Int. Cl.: A61K 49/18, A61K 41/00, B82Y 5/00, B82Y 15/00

(54) **NANOPARTICULES ULTRAFINES A MATRICE POLYORGANOSILOXANE FONCTIONNALISEE ET INCLUANT DES COMPLEXES METALLIQUES POUR LEURS APPLICATIONS EN IMAGERIE DIAGNOSTIQUE ET/OU THERAPIE**

(30) Priorité: 30.04.2010 FR 1053389
(62) Demande divisionnaire de: 11716589.4
(71) Demandeur: Institut National des Sciences Appliquées de Lyon, 69100 Villeurbanne (FR); UNIVERSITE CLAUDE BERNARD - LYON 1, 69100 Villeurbanne (FR); NANOH, 38070 Saint Quentin Fallavier (FR)
(72) Inventeur: LUX, François, 69003 LYON (FR); TILLEMENT, Olivier, 69270 FONTAINES SAINT-MARTIN (FR); SAINT JEAN, Maxime, 71100 Chalon-sur-Saone (FR); MOWAT, Pierre, 69200 VENISSIEUX (FR); PERRIAT, Pascal, 69001 LYON (FR); ROUX, Stéphane, 25440 CHENEYCEY BUILLON (FR); MIGNOT, Anna, 69100 VILLEURBANNE (FR)
(74) Mandataire: Cabinet Plasseraud

(57) **Abrégé**

L'invention concerne de nouvelles nanoparticules hybrides biocompatibles de très faible taille, utiles notamment pour le diagnostic et/ou la thérapie.

Le but de l'invention est de proposer de nouvelles nanoparticules qui soient utiles notamment comme agents de contraste en imagerie (e.g. IRM) et/ou dans d'autres techniques de diagnostic et/ou comme agents de thérapie, qui soient plus performantes que les nanoparticules de même type connues et qui combinent à la fois une petite taille (par exemple inférieure à 20 nm) et un fort taux de charge en métaux (e.g. terres rares), en particulier pour avoir en imagerie (e.g. IRM) un fort rehaussement et une réponse correcte (relaxivité accrue) aux hautes fréquences.
Ainsi, les nanoparticules selon l'invention, de diamètre d₁ entre 1 et 20 nm, comprennent chacune une matrice polyorganosiloxane (POS) incluant des cations gadolinium éventuellement associé à des cations dopants; un greffon C¹ chélatant DTPABA (dianydride d'acide diéthylènetriaminepentaacétique) lié à la matrice POS par liaison covalente -Si-C-, et présent en quantité suffisante pour pouvoir complexer tous les cations gadolinium; et éventuellement un autre greffon de fonctionnalisation Gf* lié à la matrice POS par liaison covalente -Si-C- (Gf* pouvant être issu d'un composé hydrophile (PEG); d'un composé comportant un principe actif PA1;d'un composé ciblant; d'un composé luminescent (fluorescéine).
Le procédé d'obtention de ces nanoparticules et leurs applications en imagerie et en thérapie font également partie de l'invention.

## Description

### Domaine technique

L'invention concerne de nouvelles nanoparticules hybrides biocompatibles de très faible taille, utiles notamment pour le diagnostic et/ou la thérapie.

L'invention vise également leur procédé d'obtention et leurs applications.

Dans le domaine du diagnostic, il peut s'agir de sondes pour le marquage biologique dotées de propriétés magnétiques, fluorescentes ou radioactives, d'agents de contraste pour l'imagerie par résonnance magnétique IRM, pour l'imagerie par scintigraphie SPECT (Single Photon Emission Computed Tomography), pour l'imagerie par scintigraphie PET (Positron Emission Tomography) pour l'imagerie par fluorescence, pour l'imagerie optique, pour l'imagerie par scanner X ou pour l'imagerie multimodale.

Dans le domaine thérapeutique, les utilisations envisagées sont celles d'agents radio-sensibilisants ou radioactifs pour la radiothérapie (e.g. curiethérapie), pour la neutronthérapie, d'agents pour la PDT (photodynamic therapy), d'agents de vectorisation de molécules à effet thérapeutique ou d'agents de marquage cellulaire.

Dans les deux domaines susvisés, les nanoparticules concernées peuvent être des agents de ciblage de sites thérapeutiques ou diagnostiques in vivo.

### Arrière-plan technologique

Les prérequis pour des nanoparticules appliquées au diagnostic et/ou à la thérapie, sont, entre autres :
- une forte concentration d'agents actifs (pour la détection d'un signal en tant qu'agent de contraste pour le diagnostique et/ou pour l'action thérapeutique),
- une bonne rigidité moléculaire appréciable pour une bonne vectorisation et/ou en imagerie IRM,
- une très bonne stabilité colloïdale en milieu biologique,
- une excellente biocompatibilité,
- une accessibilité (c'est-à-dire une facilité d'accès à la zone d'intérêt) et une efficacité toujours meilleure pour le ciblage thérapeutique, en particulier vis-à-vis des tumeurs,
- une bonne élimination rénale in vivo,
- une biodistribution in vivo favorable et adaptée (pas de rétention des nanoparticules par les organes ou rétention préférentielle dans les organes ciblées).

L'IRM est l'une des techniques parmi les plus utilisées pour le diagnostic médical, elle combine les avantages d'être non-invasive, rapide et sans danger pour le patient. Elle repose sur l'observation de la relaxation des protons de l'eau qui est directement dépendante des champs magnétiques (le champ magnétique important B₀ et des champs radio-fréquence), de la séquence d'impulsion, de l'environnement de l'eau dans l'organisme... L'interprétation des images donne alors accès à l'identification de la plupart des tissus. Le contraste peut être augmenté par deux types d'agents : les agents de contraste positifs T₁ et négatifs T₂.

Dans le cadre de l'invention, on s'intéresse en particulier à ces agents de contraste positifs c'est-à-dire en T₁, qui permettent un éclaircissement de l'image car le contact de l'eau avec l'agent de contraste permet de réduire le temps de relaxation longitudinal : T₁. Comme exemples d'agents de contraste T₁ utilisés en clinique, on peut citer : Gd(III)DTPA ou Gd(III)DOTA.

Les agents de contraste en T₂ sont ceux qui permettent de réduire le temps de relaxation transversal. Les nanoparticules d'oxyde de fer superparamagnétiques encapsulées sont des exemples d'agents T₂ utilisés en clinique.

Les agents de contraste en T₁ sont préférables aux agents de contraste en T₂, en raison du contraste positif qu'ils procurent. Malheureusement, la non-spécificité, le faible contraste qu'ils entraînent, leur rapide excrétion rénale et leurs propriétés dépendantes du champ restreignent encore leur application. De plus, une concentration locale en ions gadolinium souvent faible rend délicate la détection d'anomalies médicales d'une taille inférieure à quelques centimètres. Il serait donc souhaitable de pouvoir mettre en oeuvre un ciblage spécifique de sites in vivo, à l'aide d'éléments chargés en gadolinium. A ce titre, les nano-objets présentent plusieurs avantages : concentration locale en Gd plus élevée et relaxivité (r₁) par gadolinium plus importante en raison de la masse et de la rigidité plus conséquente des nanoparticules.

Au-delà de l'amélioration du contraste, on cherche également à limiter la concentration en agents de contraste dans le corps humain.

Tous ces aspects tempèrent l'utilité des composés moléculaires (les agents T₁ commerciaux présentés ci-dessus sont bien des agents moléculaires, ce n'est pas le cas des agents T₂ mais ceux-ci sont moins intéressants à cause du contraste négatif qu'ils procurent) comme agents de contraste.

Une approche multimodale est entreprise pour ces particules. Cette approche consiste à mettre en oeuvre un ensemble de nanoparticules comprenant plusieurs agents de contraste moléculaires. Elle permet de combiner non seulement différentes techniques d'imagerie, mais aussi différentes techniques de thérapie en regroupant plusieurs agents actifs en thérapie dans un même ensemble de nanoparticules. Les agents actifs en imagerie et les agents actifs en thérapie peuvent être identiques ou différents entre eux.

Cette approche semble être particulièrement adaptée pour le développement de médicaments en théranostic. On peut notamment ajouter d'autres fonctions d'imagerie (luminescence, scintigraphie...), des fonctions thérapeutiques (largage de principes actifs, radio-sensibilisation, curie-thérapie...) ainsi que des fonctions de ciblage biologique pour une concentration des agents thérapeutiques dans la zone d'intérêt.

Les composés moléculaires, de par leur faible taille, ne permettent en général pas de combiner plusieurs agents actifs, c'est-à-dire plusieurs propriétés au sein d'un même ensemble (manque de place au niveau d'un composé moléculaire).

FR-2867180-B1 décrit des nanoparticules sondes hybrides pour le marquage biologique (fluorescence-luminescence-radioactivité), pour la détection (reconnaissance) ou le suivi de substances spécifiques, appelées cibles. De telles sondes sont particulièrement utilisées pour la cytométrie de flux, l'histologie, les tests immunologiques ou la microscopie de fluorescence, aussi bien pour l'étude de matériaux biologiques que de matériaux non biologiques. Les nanoparticules selon le FR-2867180-B1 ont une structure coeur/coquille (*core* / *shell*) dont le coeur est composé à au moins 90% en poids, de Gd₂O₃ c'est-à-dire d'un oxyde de terre rare éventuellement dopé avec une terre rare ou un actinide, et dont la coquille est un enrobage constitué majoritairement de polysiloxane obtenu par hydrolyse/condensation sol/gel d'aminopropyltriéthoxysilane (APTES) et de tétraéthoxysilane (TEOS) en présence de triéthylamine. Le diamètre de cette structure coeur/coquille est par exemple de 8,5 ; 11,5 ou 9,2 nm. La coquille polyorganosiloxane (POS) peut être fonctionnalisée c'est-à-dire greffée par un colorant marqueur (fluorescéine) ou un ligand biologique tel qu'un acide nucléique.

FR-2922106-A1 décrit l'utilisation de nanoparticules hybrides radio-sensibilisantes, à forte concentration en oxydes de lanthanides, comme radio-sensibilisants qui agissent en combinaison avec la radiation pour induire une réponse plus efficace et augmenter l'efficacité thérapeutique. Ces nanoparticules ont une structure coeur/coquille dont le coeur (diamètre e.g. 1,5 nm) est composé de Gd₂O₃ (éventuellement associé en mélange 50/50 à de l'oxyde d'holmium), et dont la coquille est un enrobage constitué majoritairement de polysiloxane obtenu par hydrolyse/condensation sol/gel d'aminopropyltriéthoxysilane (APTES) et de tétraéthoxysilane (TEOS) en présence de triéthylamine. Le diamètre de cette structure coeur/coquille est par exemple de 2,7 nm. La coquille POS est ensuite fonctionnalisée c'est-à-dire greffée soit au moyen d'un polyol tel que le polyéthylèneglycol, soit au moyen d'un chélatant e.g. l'acide diéthylènetriamine pentaacétique (DTPA). Ce type de nanoparticule coeur/coquille fonctionnalisée a par exemple un diamètre total de 11,7 nm.

FR-2930890-A1 divulgue de nouveaux agents de radiothérapie ciblée ou de curiethérapie (radionucléides) constitués par des nanoparticules à forte concentration en oxyde ou oxohydroxyde de terres rares radioactives. Ces nanoparticules sont par exemple des nanoparticules constituées d'un coeur d'oxyde d'holmium enrobé de polysiloxane fonctionnalisé. Le coeur est obtenu par dissolution de sels de chlorure d'holmium dans du diéthylène-glycol. La taille de ce coeur est d'environ 1,5 nm. La couche de polysiloxane fonctionnalisé d'une épaisseur de 0,5 nm est synthétisée par voie sol-gel, à partir principalement d'APTES et de TEOS. Les particules ainsi enrobées sont ensuite fonctionnalisées soit par du PEG250, soit par de l'acide diéthylènetriaminepentaacétique dianhydre (DTPABA). La suspension ainsi préparée est lyophilisée. Les tailles hydrodynamiques sont pour le coeur : 1,5 nm ; après enrobage : 3,1 nm et après fonctionnalisation par le DTPABA : 3,6 nm ; après lyophilisation : 4,0 nm.

Les nanoparticules selon ces trois documents FR-2867180-B1, FR-2922106-A1 et FR-2930890-A1 ont notamment comme intérêt, leur coquille polysiloxane qui améliore leur biocompatibilité. Leur petite taille est en outre un atout au regard de l'élimination rénale (clearance) et de la biodistribution in vivo. Mais, l'élément actif comme agent de contraste T1 en IRM dans ces nanoparticules, à savoir par exemple le gadolinium présent au centre du coeur d'oxyde de lanthanide est nettement moins accessible par l'eau que celui en surface ou que celui qui serait complexé par le DTPA ou le DOTA à l'intérieur ou en surface de la couche de polysiloxane. Ceci entraîne une efficacité moindre de l'objet par atome de gadolinium pour ces nanoparticules de type coeur/coquille. En outre, s'il est possible d'atteindre des diamètres de moins de 10 nm pour ces nanoparticules à coeur d'oxyde de gadolinium, par exemple, cela se fait en diminuant la taille de la couche externe de polysiloxane. Il peut en résulter des problèmes de dissolution du coeur lié à la plus faible protection apportée par une couche réduite de polysiloxane. Ceci entraîne des risques de toxicité liés aux concentrations locales élevées en gadolinium.

WO-2007124131-A divulgue des nanoparticules hybrides utiles comme agents multimodaux de contraste en imagerie notamment IRM. Ces nanoparticules hybrides comprennent une matrice polymère inorganique à base de silice (ou organique: polyacrylate-polylactide) et une pluralité de complexes de coordination comportant chacun un groupement chélatant fonctionnalisé (DTTA-DTPA-DOTA), un ion métallique paramagnétique (lanthanide/actinide: Gd- Mn), un luminophore (fluorescéine). Le radical de fonctionnalisation est par exemple un aminopropyl-(trimethoxysilyl).

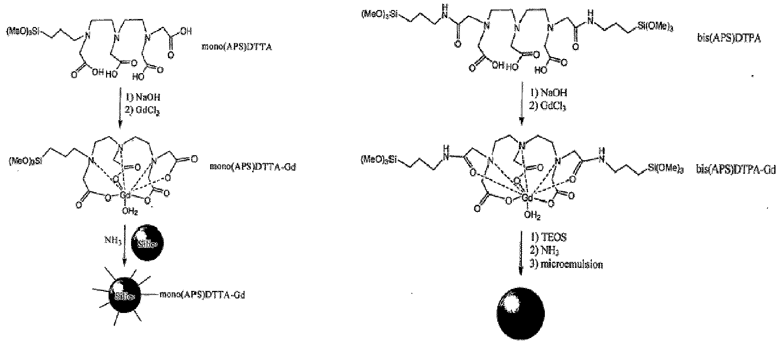

Les nanoparticules de silice ainsi obtenues ont un diamètre de l'ordre de 40 nm. Ces nanoparticules n'ont pas une structure coeur/coquille et donc, a priori n'ont pas les inconvénients y afférents. Cependant, leur taille de plusieurs dizaines de nanomètres est un handicap sérieux en termes d'élimination in vivo.

L'art antérieur ne divulgue pas des nanoparticules alliant une très petite taille, par exemple inférieure à 20 nm, et un fort taux de charge en agent de contraste, e.g. T1 pour l'IRM (gadolinium).

La très faible taille des nanoparticules est primordiale pour obtenir in vivo une bonne élimination rénale et une biodistribution adaptée.

Le taux de charge élevé en complexes magnétiques combiné à la taille et à la rigidité de l'objet permet d'obtenir un r₁ par objet et par atome de gadolinium important (supérieur à celui de l'ion libre et à fortiori des composés moléculaires) ce qui est requis pour avoir en imagerie (e.g. IRM) un fort rehaussement et une réponse correcte aux hautes fréquences.

### Objectifs et brève description de l'invention

La présente invention vise à satisfaire au moins l'un des objectifs suivants :
(i) proposer de nouvelles nanoparticules qui soient utiles notamment comme agents de contraste en imagerie (e.g. IRM) et/ou dans d'autres techniques de diagnostic et/ou comme agents de thérapie, et qui soient plus performantes que les nanoparticules de même type connues ;
(ii) proposer de nouvelles nanoparticules à base de polymère silicique, de métaux (e.g. terres rares) et de chélatants, qui soient utiles notamment comme agents de contraste en imagerie (e.g. IRM) et/ou dans d'autres techniques de diagnostic et/ou comme agents de thérapie, et qui combinent à la fois une petite taille (par exemple inférieure à 20 nm) et un fort taux de charge en métaux (e.g. terres rares), en particulier pour avoir en imagerie (e.g. IRM) un fort rehaussement (lié par exemple à une forte relaxivité r₁) et une réponse correcte (relaxivité accrue) aux hautes fréquences ;
(iii) proposer de nouvelles nanoparticules à base de polymère silicique, de métaux (e.g. terres rares) et de chélatants, qui soient utiles notamment comme agents de contraste en imagerie (e.g. IRM) et/ou dans d'autres techniques de diagnostic et/ou comme agents de thérapie, et qui aient une bonne rigidité moléculaire appréciable en imagerie IRM ;
(iv) proposer de nouvelles nanoparticules à base de polymère silicique, de métaux (e.g. terres rares) et de chélatants, qui soient utiles notamment comme agents de contraste en imagerie (e.g. IRM) et/ou dans d'autres techniques de diagnostic et/ou comme agents de thérapie, et qui aient une excellente biocompatibilité ;
(v) proposer de nouvelles nanoparticules à base de polymère silicique, de métaux (e.g. terres rares) et de chélatants, qui soient utiles notamment comme agents de contraste en imagerie (e.g. IRM) et/ou dans d'autres techniques de diagnostic et/ou comme agents de thérapie, et qui aient une très bonne stabilité colloïdale en milieu biologique ;
(vi) proposer de nouvelles nanoparticules à base de polymère silicique, de métaux (e.g. terres rares) et de chélatants, qui soient utiles notamment comme agents de contraste en imagerie (e.g. IRM) et/ou dans d'autres techniques de diagnostic et/ou comme agents de thérapie, et qui aient une accessibilité (la petite taille des particules leur permet d'accéder plus facilement aux zones d'intérêt) et une efficacité toujours meilleure pour le ciblage thérapeutique, en particulier vis-à-vis des tumeurs ;
(vii) proposer de nouvelles nanoparticules à base de polymère silicique, de métaux (e.g. terres rares) et de chélatants, qui soient utiles notamment comme agents de contraste en imagerie (e.g. IRM) et/ou dans d'autres techniques de diagnostic et/ou comme agents de thérapie, et qui aient une forte concentration d'actifs ;
(viii) proposer de nouvelles nanoparticules à base de polymère silicique, de métaux (e.g. terres rares) et de chélatants, qui soient utiles notamment comme agents de contraste en imagerie (e.g. IRM) et/ou dans d'autres techniques de diagnostic et/ou comme agents de thérapie, et qui montrent in vivo une bonne élimination rénale (clearance) et une biodistribution favorable et adaptée ;
(ix) proposer de nouvelles nanoparticules à base de polymère silicique, de métaux (e.g. terres rares) et de chélatants, qui soient utiles notamment comme agents de contraste en imagerie (e.g. IRM) et/ou dans d'autres techniques de diagnostic et/ou comme agents de thérapie, et qui soient utilisables comme une plateforme permettant de combiner de nombreuses fonctionnalités (en plus de celles en IRM) : fonctions d'imagerie (fluorescence, scintigraphie...) ou thérapeutiques (radiosensibilisation, curie-thérapie, photothérapie dynamique...) ;
(x) proposer de nouvelles nanoparticules à base de polymère silicique, de métaux (e.g. terres rares) et de chélatants, qui soient utiles notamment comme agents de contraste en imagerie (e.g. IRM) et/ou dans d'autres techniques de diagnostic et/ou comme agents de thérapie, et dont la toxicité liée aux métaux (e.g. terres rares) qu'elle contient soit réduite ;
(xi) proposer un nouveau procédé d'obtention de nanoparticules telles que celles visées dans les objectifs (i) à (x) ci-dessus ;
(xii) proposer de nouvelles suspensions de nanoparticules telles que celles visées dans les objectifs (i) à (x) ci-dessus ;
(xiii) proposer de nouvelles compositions de diagnostic, de contraste en imagerie (e.g. IRM), de thérapie ou de marquage cellulaire, à base des nanoparticules telles que celles visées dans les objectifs (i) à (x) ci-dessus.

Ces objectifs, parmi d'autres, sont atteints par la présente invention qui concerne dans un premier de ses aspects, des nanoparticules caractérisées
(i) en ce qu'elles comprennent chacune
   - une matrice polyorganosiloxane (POS) incluant des cations (e.g.métalliques) Mⁿ⁺ (n=2 à 6) de préférence de terre rare, éventuellement en partie sous forme d'un oxyde et/ou d'un oxohydroxyde métallique (M), éventuellement associé à des cations dopants D^{m+} (m=2 à 6), de préférence une terre rare différente de M, un actinide et/ou un élément de transition ;
   - un greffon de fonctionnalisation C¹ chélatant qui est :
      * issu d'un chélatant C1,
      * lié à la matrice POS par liaison covalente -Si-C-,
      * et en quantité suffisante pour pouvoir complexer tous les cations Mⁿ⁺ et D^{m+} ; le greffon C¹ étant de préférence en excès par rapport aux cations Mⁿ⁺ et D^{m+} ; le chélatant C1 duquel le greffon C¹ est issu correspondant à une ou plusieurs espèces différentes ;
   - éventuellement un autre greffon de fonctionnalisation Gf* lié à la matrice POS par liaison covalente -Si-C-, Gf* pouvant être issu :
      * d'un composé hydrophile (PEG),
      * d'un composé comportant un principe actif PA1,
      * d'un composé ciblant,
      * d'un composé luminescent (e.g. fluorescéine) ;
(ii) en ce que leur diamètre d₁ est compris entre 1 et 20 nm, de préférence entre 1 et 10 nm ;
(iii) et éventuellement en ce qu'elles sont porteuses d'un principe actif PA2, identique ou différent à PA1.

Dans un deuxième aspect, l'invention vise un procédé d'obtention des nanoparticules selon l'une au moins des revendications précédentes caractérisé en ce qu'il comprend les étapes suivantes :
a Synthèse de coeurs à base d'un oxyde et/ou un oxohydroxyde métallique (M), de préférence de terre rare, au moins en partie sous forme cationique Mⁿ⁺ (n=2 à 6), éventuellement dopé par un dopant (D), de préférence une terre rare différente de M, un actinide et/ou un élément de transition ;
   cette synthèse consistant essentiellement à mélanger une base (de préférence une base forte telle que NaOH) avec un sel de M dissous dans un solvant de préférence choisi dans le groupe comprenant les alcools (avantageusement le DEG) ;
b Enrobage des coeurs de l'étape (a) par du polyorganosiloxane (POS) consistant essentiellement à mettre en oeuvre une technique sol/gel d'hydrolyse-condensation d'espèces siliciques et d'alcoxysilanes, en présence d'un base ou d'un acide et éventuellement d'un principe actif PA1 et/ou PA2 ;
c Surenrobage de fonctionnalisation des coeurs enrobés de l'étape (b) consistant essentiellement à mettre en présence ces coeurs enrobés de l'étape (b) en présence d'un précurseur de greffons de fonctionnalisation C¹, (de préférence issu d'un chélatant C1 choisi parmi les produits définis ci-après), et éventuellement en présence d'un greffon de fonctionnalisation Gf* (de préférence un composé hydrophile et/ou un composé comportant un principe actif PA1 et/ou un composé ciblant biologique et/ou un composé luminescent choisi parmi les produits définis ci-après) ;
d Purification des nanoparticules surenrobées/fonctionnalisées de diamètre d₀, de préférence par filtration tangentielle, dialyse et/ou par précipitation/lavage ;
e Dissolution des coeurs M des nanoparticules surenrobées/fonctionnalisées de l'étape (c) consistant essentiellement à les mettre en présence d'un agent modificateur du pH et/ou d'un chélatant C2 apte à complexer tout ou partie des cations Mⁿ⁺ et D^{m+}, de sorte que le diamètre d₀ des nanoparticules est réduit à une valeur d₁ comprise entre 1 et 20 nm, de préférence entre 1 et 10 nm; ce chélatant C2 étant choisi parmi les produits chélatants définis ci-après ;
f Eventuel ajout d'un sel cationique destiné à être au moins en partie complexé par le chélatant C¹ ;
les étapes (c), (d), (e), (f) pouvant être réalisées dans un ordre différent ou en même temps. Dans le cas où tout le coeur aurait été dissous lors de l'étape (c), l'étape (e) devient facultative.

Dans un troisième aspect, l'invention vise une suspension de nanoparticules et l'extrait sec d'une telle suspension.

Dans un quatrième aspect, l'invention vise un liquide injectable comprenant ces nanoparticules.

Dans un cinquième aspect, l'invention vise :
→ une composition d'aide au diagnostic, de préférence composition de contraste,
→ une composition thérapeutique,
→ ou une composition de marquage cellulaire,
caractérisée en ce qu'elle comprend des nanoparticules selon l'invention et/ou obtenues par le procédé selon l'invention et/ou de la suspension selon l'invention et/ou préparé à partir de la matière solide selon l'invention.

### Définitions

Dans tout ce texte, tout singulier désigne indifféremment un singulier ou un pluriel. Les définitions des termes utilisés dans le cadre du présent exposé et énoncées ci-dessous, correspondent à une terminologie selon l'invention et ne sont données qu'à titre non limitatif.
∘ APTES désigne le (3-aminopropyl)triethoxysilane.
∘ BAPTA désigne l'acide 1,2 Bis-2-aminophénoxyéthane-N,N,N',N'-tétraacétique.
∘ DEG désigne le diéthylène glycol.
∘ DOTA désigne l'acide 1,4,7,10-tétraazacyclododécane-1,4,7,10-tétraacétique.
∘ DOTA-NHS désigne l'acide 1,4,7,10-tétraazacyclododécane-1,4,7,10-tétraacétique N-hydroxysuccinimide.
∘ DTPA désigne l'acide diéthylènetriaminepentaacétique.
∘ DTPABA désigne le dianhydride d'acide diéthylènetriaminepentaacétique.
∘ DTTA désigne l'acide diéthylènetriamine tétraacétique.
∘ EDTA désigne l'acide éthylènediamine tétraacétique.
∘ EGTA désigne l'acide éthylène glycol-acide bis(2-aminoethyléther)-N,N,N',N'-tétraacétique
∘ EXAFS désigne : "Extended X-Ray Absorption Fine Structure".
∘ IRM désigne : "Imagerie par Résonance Magnétique".
∘ NOTA désigne l'acide 1,4,7-triazacyclononane-N,N',N"-triacétique.
∘ PDT désigne : "photodynamic therapy"
∘ PET désigne : "Positron Emission Tomography" ou scintigraphie PET.
∘ PEG désigne le polyéthylène glycol.
∘ POS désigne le polyorganosiloxane.
∘ PPG désigne le polypropylène glycol.
∘ SPECT désigne : "Single Photon Emission Computed Tomography" ou scintigraphie SPECT
∘ TEOS désigne le tétraéthoxysilane.
∘ TEM désigne la microscopie électronique en transmission.
∘ XPS désigne : "X-ray photoelectron spectroscopy".
∘ Un composé *"hydrophile"* désigne un composé ayant une forte affinité pour l'eau, par *"forte"* on entend par exemple un composé qui puisse se dissoudre ou se disperser de façon homogène dans l'eau à température ambiante à plus de 100 grammes par litre de solution.
∘ *"alkyle",* désigne une chaîne hydrocarbonée saturée, linéaire ou ramifiée, éventuellement substituée (e.g. par un ou plusieurs alkyles), de préférence de 1 à 10 atomes de carbone, par exemple de 1 à 8 atomes de carbone, mieux encore de 1 à 7 atomes de carbone.
   Des exemples de groupements alkyle sont notamment méthyle, éthyle, isopropyle, n-propyle, tert-butyle, isobutyle, n-butyle, n-pentyle, isoamyle et 1,1-diméthylpropyle.
   La partie alkyle du groupement alcoxy est telle que définie ci-dessus.
∘ *"cycloalkyle",* désigne un groupement hydrocarboné saturé mono- ou polycyclique, de préférence mono- ou bicyclique, présentant préférablement de 3 à 10 atomes de carbone, mieux encore de 3 à 8.
∘ *"groupement hydrocarboné saturé polycyclique",* désigne un groupement présentant deux ou plusieurs noyaux cycliques rattachés les uns aux autres par des liaisons π ou/et condensés deux à deux.
   Des exemples de groupements cycloalkyle polycycliques sont adamantane et norbornane.
   Des exemples de groupements cycloalkyle monocycliques sont cyclopropyle, cyclobutyle, cyclopentyle, cyclohexyle, cycloheptyle et cyclooctyle.
∘ *"aryle",* désigne un groupement hydrocarboné aromatique, ayant de 6 à 18 atomes de carbone, monocyclique ou polycyclique et de préférence monocyclique ou bicyclique. Il doit être entendu que, dans le cadre de l'invention, par *"groupement aromatique polycyclique",* on entend un groupement présentant deux ou plusieurs noyaux aromatiques, condensés (orthocondensés ou ortho et péricondensés) les uns aux autres, c'est-à-dire présentant, deux à deux, au moins deux carbones en commun.
   Ledit groupement hydrocarboné aromatique ("*aryle*") est éventuellement substitué par exemple par un ou plusieurs alkyles en C1-C3, un ou plusieurs groupements hydrocarbonés halogénés (e.g. CF3), un ou plusieurs alcoxy (e.g. CH3O) ou un ou plusieurs groupements hydrocarbonés comprenant un ou plusieurs motifs cétone (e.g. CH3CO-).
   A titre d'exemple d'aryle, on peut mentionner les radicaux phényle, naphtyle, anthryle et phénanthryle. phényle ; p-chlorophényle ; m-chlorophényle ; dichloro-3,5-phényle ; trichlorophényle ; tétrachlorophényle ; o-, p- ou m-tolyle ; α,α,α-trifluorotolyle ; xylyles comme diméthyle-2,3 phényle ; diméthyle-3,4-phényle. Ces groupements peuvent être éventuellement halogénés, ou bien encore être choisis parmi les radicaux cyanoalkyles.
   Les halogènes sont par exemple le fluor, le chlore, le brome et l'iode, de préférence le chlore ou le fluor.
∘ *"arylalkyle"* désigne un groupement alkyle tel que défini ci-dessus, substitué par un ou plusieurs groupements aryle sur sa chaîne hydrocarbonée, le groupement aryle étant tel que défini ci-dessus. Des exemples en sont benzyle et triphénylméthyle.
∘ *"alcényle"* désigne une chaîne hydrocarbonée insaturée, linéaire ou ramifiée, substituée ou non, présentant au moins une double liaison oléfinique, et plus préférablement une seule double liaison. De préférence, le groupement "alcényle" présente de 2 à 8 atomes de carbone, mieux encore de 2 à 6. Cette chaîne hydrocarbonée comprend éventuellement au moins un hétéroatome tel que O, N, S. Des exemples préférés de groupements alcényle sont les groupements vinyle, allyle et homoallyle.
∘ *"alcynyle",* désigne selon l'invention, une chaîne hydrocarbonée insaturée, linéaire ou ramifiée, substituée ou non, présentant au moins une triple liaison acétylénique, et plus préférablement une seule triple liaison. De préference, le groupement alcynyle présente de 2 à 8 atomes de carbone, mieux encore de 2 à 6 atomes de carbone. A titre d'exemple, on peut citer le groupement acétylényle, ainsi que le groupement propargyle. Cette chaîne hydrocarbonée comprend éventuellement au moins un hétéroatome tel que O, N, S.

### Description détaillée de l'invention

### Nanoparticules de l'invention

Les nanoparticules selon l'invention peuvent être définies par leur structure matricielle de POS incluant une fonctionnalisation chélatante C¹ issue d'un chélatant C1 et conduisant à la formation de complexes [Mⁿ⁺]/[C¹] voire [D^{m+}]/[C¹], et, selon une modalité facultative mais néanmoins préférée, des greffons de fonctionnalisation Gf* par exemple issus :
∘ d'un composé hydrophile (PEG) ;
∘ d'un composé comportant un principe actif PA1 ;
∘ d'un composé ciblant ;
∘ et/ou d'un composé luminescent (fluorescéine).

Les nanoparticules selon l'invention peuvent également être définies par leur mode d'obtention. Les nanoparticules ainsi définies constituent en elles-mêmes un autre objet de l'invention et sont caractérisées en ce que la matrice POS de chaque nanoparticule est obtenue à partir d'une nanoparticule de diamètre d₀ comprenant :
- un coeur à base d'un oxyde et/ou un oxohydroxyde métallique (M), de préférence de terre rare, au moins en partie sous forme cationique Mⁿ⁺ (n=2 à 6), éventuellement dopé par un dopant (D), de préférence une terre rare différente de M, un actinide et/ou un élément de transition ;
- au moins une couche d'enrobage à base de POS ;
- et, éventuellement un surenrobage à base d'un agent de fonctionnalisation, de préférence choisi parmi les chélatants de fonctionnalisation C1 aptes à séquestrer des cations et/ou les composés ciblants et/ou les composés comportant un principe actif PA1 et/ou les composés hydrophiles et/ou les composés luminescents ;
ladite nanoparticule étant soumise à une dissolution du coeur M, de préférence à l'aide d'un agent modificateur du pH et/ou d'un chélatant C2, identique ou différent à C1, apte à complexer tout ou partie des cations Mⁿ⁺ et D^{m+}, de sorte que le diamètre d₀ de la nanoparticule est réduit à une valeur d₁ comprise entre 1 et 20 nm, de préférence entre 1 et 10 nm.

Ces nanoparticules ont donc pour particularité d'être "sans coeur", ou plus précisément de ne plus présenter de coeur. Elles ne comprennent pas de coeur cristallisé. Plus précisément encore Elles ne comprennent pas de coeur encapsulé par au moins un enrobage.

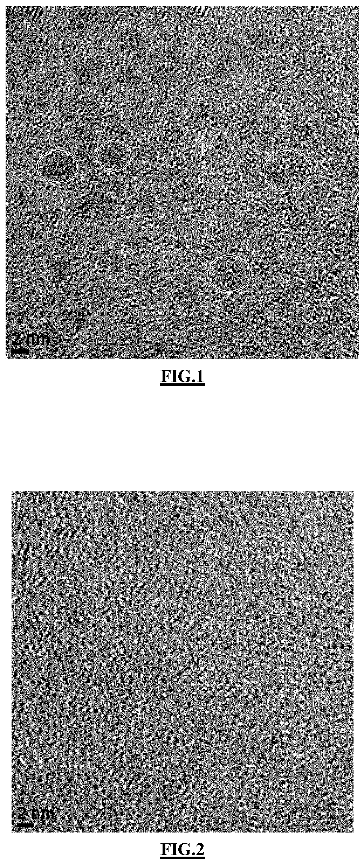

Pour illustrer cela, on se référera à titre non limitatif aux figures 1 et 2 annexées. La figure 1 est une image en TEM (grossissement du microscope x600 000) de particules précurseur des nanoparticules selon l'invention. Ces particules précurseur sont formées par des coeurs d'oxyde métallique M (oxyde de gadolinium), enrobés de polysiloxane, et fonctionnalisés par un complexant C¹ (e.g. DOTA anhydre). Les tailles observées sont comprises entre 3 et 5 nm.

Les molécules de DOTA ne sont pas visibles au TEM, on ne voit que le coeur enrobé. La figure 2 annexée est une image en TEM après dissolution des coeurs, prise dans les mêmes conditions que l'image de la figure 1. On n'observe aucune particule qui comporterait un coeur, alors que l'analyse chimique locale indique la présence de gadolinium dans la zone observée.

Une caractéristique essentielle de ces particules ultrafines vient de leur très petite taille, de l'ordre de quelques nanomètres. A ces très petites tailles (inférieures ou égales à, dans un ordre croissant de préférence 20; 18; 15; 12; 10; 8; 5; 3 nanomètres), les particules présentent un comportement quasi moléculaire, permettant :
→ une très bonne stabilité colloïdale en milieu biologique,
→ une bio-distribution favorable avec une possibilité d'élimination par les voies naturelles adaptées, c'est à dire une bio-distribution rénale sans captation dans les organes vitaux, tout en présentant un temps de rétention plus long que celui de complexes moléculaires, leur aspect plus massif permettant en outre d'éviter une diffusion trop importante,
→ une meilleure accessibilité et efficacité pour le ciblage tumoral,
→ une possibilité d'insérer une forte concentration d'élément actifs, c'est-à-dire obtenir un grand taux de chélatants C1 greffées sur et au sein de la matrice polysiloxane et obtenir un rapport C1 sur silicium important, avantageusement supérieur ou égal à, dans un ordre croissant de préférence, 1; 5; 10; 20; 30; 40; 50 % en masse. Pour la recherche d'un signal donné ou d'un effet thérapeutique lié à l'élément complexé, ceci limite alors l'injection de l'élément exogène silicium.

S'agissant de la taille, il peut être observé que ces nanoparticules sont également caractérisées par leur masse molaire (en kDa) supérieure ou égale à, dans un ordre croissant de préférence, 2, 3, 5, et inférieure ou égale à, dans un ordre croissant de préférence, 200, 100, 50, 20, 10.

Leurs très bonnes stabilités et compatibilités chimiques sont deux caractéristiques très importantes de ces nanoparticules. Leur squelette de base est formé de POS, c'est-à-dire majoritairement de composés comprenant Si, C, H, O et N, base des silicones, composés réputés pour leur très bonne compatibilité biologique et leur faible toxicité. Les éléments actifs supplémentaires, comme les cations métalliques sont eux insérés au sein des particules par complexation grâce à des molécules stables et reconnues fiables pour les applications biologiques. La concentration résiduelle en ions libres peut alors être contrôlée et maintenue bien en dessous des seuils de toxicité critiques. Les échanges de cations complexés avec des cations endogènes peuvent être également contrôlés par utilisation au sein d'une même particule d'une partie des fonctions chélatantes C¹. La possibilité d'ajout sur les nanoparticules de complexant libres (e.g. C2) de cations métalliques permet d'assurer que la libération accidentelle de M (e.g. du gadolinium) voire de D, puisse être immédiatement compensée par la complexation par un autre chélatant réduisant ainsi les risques de toxicité liée à la nature du gadolinium.

Ces nanoparticules de POS "sans coeur" comprennent une forte densité de greffons chélatants C¹ liés (ou non C2) de façon covalente au squelette (matrice) de POS.

De préférence, les chélatants C1 et/ou C2 sont présents dans les nanoparticules en quantité telle que tout ou partie des ions, en particulier les cations Mⁿ⁺ voire D^{m+} ne sont pas libres mais sont complexés. Mieux encore, le pourcentage massique des éléments M et D impliqués dans des liaisons M-O-M ou M-O-D ou D-O-D ou M-O-Si ou D-O-Si, par rapport au nombre total d'éléments M et D, inférieur ou égal à, dans un ordre croissant de préférence 10; 5; 2, 1; 0,5; 10⁻¹; 10⁻²; 10⁻³.

Le nombre de chélatants C¹ et/ou C2 dans les nanoparticules est supérieur au nombre de cations restants Mⁿ⁺ et/ou de D^{m+}, voire d'autres cations (e.g. Ca⁺⁺, Mg⁺) éventuellement ajoutés en pus des Mⁿ⁺ et/ou de D^{m+}.

Ce pourcentage de liaisons (oxydes) M-O-M ou M-O-D ou D-O-D ou M-O-Si ou D-O-Si peut être mesuré par les techniques connues de type EXAFS, XPS, spectroscopie vibrationnelle, microscopie électronique en transmission couplée à des analyses structurales etc.

Cette mesure permet d'évaluer le nombre de liaisons spécifiques du coeur, cette mesure permet une mesure quantitative de la présence ou non du coeur, elle permet aussi d'évaluer les espèces M ou D qui pourraient facilement se dissoudre et se retrouver sous forme ionique en solution.

Les chélatants C1 peuvent être greffés en surface des particules de polysiloxane ou directement insérés au sein de la matrice POS. Une partie ou la totalité de ces chélatants sont destinés à complexer des cations Mⁿ⁺ (e.g. du gadolinium) voire D^{m+}. Une autre partie de ces chélatants peut servir à la complexation de cations endogènes pour assurer une bonne compatibilité avec les milieux biologiques rencontrés.

De préférence, au moins 1% et de préférence au moins 10% des chélatants C¹ ne sont pas complexés par des cations Mⁿ⁺, voire ni D^{m+}

L'un des intérêts majeurs des nanoparticules selon l'invention, est que M et /ou D puisse être un agent actif en imagerie (e.g. un agent de contraste) et/ou en thérapie.

En effet, M/Mⁿ⁺ (e.g. du gadolinium) voire D/D^{m+} présentent des propriétés remarquables pour des applications biologiques en imagerie et/ou en thérapie, comme par exemple des propriétés magnétiques, fluorescentes, radioactives (élément de numéro atomique élevé) ou radiosensibilisantes (X, gamma, béta, neutron).

Ces nanoparticules peuvent donc être utilisées comme agent de contraste dans des systèmes d'imagerie comme : l'IRM, la scintigraphie SPECT, la scintigraphie PET, l'imagerie par fluorescence, les scanners X.

Outre la fonctionnalisation chélatante C¹, ces nanoparticules peuvent être modifiées (fonctionnalisation) en surface par des composés hydrophiles (PEG) et/ou chargées différemment pour adapter leur bio-distribution au sein de l'organisme et/ou permettre un bon marquage cellulaire, en particulier pour le suivi des thérapies cellulaires.

En outre, elles peuvent être fonctionnalisées en surface par des composés ciblants biologiques, pour accéder préférentiellement à certaines zones d'intérêt de l'organisme, en particulier aux zones tumorales. De cette façon, on concentre l'agent porté par ces nanoparticules, dans la zone d'intérêt sans avoir à augmenter de manière très importante les quantités injectées comme c'est le cas actuellement.

La fonctionnalisation peut se faire également par des composés comportant un principe actif PA1 et/ou des composés luminescents (fluorescéine)

Il en résulte des possibilités d'utilisations thérapeutiques comme agent radiosensibilisant en combinaison avec des radiothérapies, des neutronthérapies, comme agent radioactif pour des traitements de curiethérapie, comme agent pour la PDT (photodynamic therapy) ou comme agent de vectorisation de molécules à effet thérapeutique.

Une caractéristique essentielle de ces particules ultrafines vient de leur très petite taille, de l'ordre de quelques nanomètres. A ces très petites tailles (inférieures ou égales à, dans un ordre croissant de préférence 20; 18; 15; 12; 10; 8; 5; 3 nanomètres), les particules présentent un comportement quasi moléculaire, permettant :
→ une très bonne stabilité colloïdale en milieu biologique,
→ une bio-distribution favorable avec une possibilité d'élimination par les voies naturelles adaptées, c'est à dire une bio-distribution rénale sans captation dans les organes vitaux, tout en présentant un temps de rétention plus long que celui de complexes moléculaires, leur aspect plus massif permettant en outre d'éviter une diffusion trop importante,
→ une meilleure accessibilité et efficacité pour le ciblage tumoral,
→ une possibilité d'insérer une forte concentration d'élément actifs, c'est-à-dire obtenir un grand taux de chélatants C1 greffées sur et au sein de la matrice polysiloxane et obtenir un rapport C1 sur silicium important, avantageusement supérieur ou égal à, dans un ordre croissant de préférence, 1; 5; 10; 20; 30; 40; 50% en masse. Pour la recherche d'un signal donné ou d'un effet thérapeutique lié à l'élément complexé, ceci limite alors l'injection de l'élément exogène silicium.

S'agissant de la taille, il peut être observé que ces nanoparticules sont également caractérisées par leur masse molaire (en kDa) supérieure ou égale à, dans un ordre croissant de préférence, 2, 3, 5, et inférieure ou égale à, dans un ordre croissant de préférence, 200, 100, 50, 20, 10.

Leurs très bonnes stabilités et compatibilités chimiques sont deux caractéristiques très importantes de ces nanoparticules. Leur squelette de base est formé de POS, c'est-à-dire majoritairement de composés comprenant Si, C, H, O et N, base des silicones, composés réputés pour leur très bonne compatibilité biologique et leur faible toxicité. Les éléments actifs supplémentaires, comme les cations métalliques sont eux insérés au sein des particules par complexation grâce à des molécules stables et reconnues fiables pour les applications biologiques. La concentration résiduelle en ions libres peut alors être contrôlée et maintenue bien en dessous des seuils de toxicité critiques. Les échanges de cations complexés avec des cations endogènes peuvent être également contrôlés par utilisation au sein d'une même particule d'une partie des chélatants C1. La possibilité d'ajout sur les nanoparticules de complexants libres de cations métalliques permet d'assurer que la libération accidentelle de M (e.g. du gadolinium) voire de D, puisse être immédiatement compensée par la complexation par un autre chélatant réduisant ainsi les risques de toxicité liée à la nature du gadolinium.

Une autre caractéristique de ces nanoparticules est le maintien du caractère rigide des objets et de la géométrie globale des particules après injection. Cette forte rigidité tridimensionnelle est assurée par la matrice polysiloxane, où la majorité des siliciums sont liés à 3 ou 4 autres atomes de silicium via un pont oxygène. La combinaison de cette rigidité avec leur petite taille permet d'augmenter la relaxivité de ces nanoparticules pour les fréquences intermédiaires (20 à 60 MHz) par rapport aux composés commerciaux (complexes à base de Gd-DOTA par exemple), mais aussi pour des fréquences supérieures à 100 MHz présentes dans les IRM haut champ de nouvelle génération.

Cette rigidité, non présente dans les polymères, est aussi un atout pour la vectorisation et l'accessibilité des molécules ciblantes.

Par ailleurs, il doit être souligné que la biocompatibilité de ces nanoparticules n'est pas la moindre de leurs qualités.

### Formule générale

Les nanoparticules selon l'invention sont également caractérisées par la formule générale suivante:

**[C¹]ₐ[R]_{b}Si[O]_{c}[OH]_{d}[Mⁿ⁺]ₑ[D^{m+}]_{f}[Gf*]_{g}**

avec :
◆ C¹ sont des greffons de fonctionnalisation formés par des radicaux hydrocarbonés monovalents, identiques ou différents entre eux, chélatants et reliés chacun au Si par une liaison Si-C ;
◆ R sont des radicaux monovalents, identiques ou différents entre eux, constituant un greffon de fonctionnalisation relié au Si par une liaison Si-C, et comprenant de préférence un groupe hydrophile et au moins un atome N ou O ;
◆ Mⁿ⁺ sont des cations métalliques identiques ou différents entre eux avec n=2 à 6 ;
◆ D^{m+} sont des cations métalliques identiques ou différents entre eux avec m=2 à 6 ;
◆ Gf* sont des greffons de fonctionnalisation autres que C¹ et formés par des radicaux hydrocarbonés monovalents, identiques ou différents entre eux, reliés chacun au Si par une liaison Si-C et pouvant être issu :
   ∘ d'un composé hydrophile (PEG) ;
   ∘ d'un composé comportant un principe actif PA1;
   ∘ d'un composé ciblant;
   ∘ d'un composé luminescent (fluorescéine) ;
◆ a supérieur ou égal à 0,01 et inférieur ou égal à 0,8 ; avec de préférence a ≥ d + e;
◆ b inférieur ou égal à 0,7 ;
◆ c supérieur ou égal à 0,5 et inférieur à 1,9 ;
◆ g supérieur ou égal à 0 et inférieur à 0,3 ;
◆ e+f supérieur ou égal à 0,01 et inférieur ou égal à 0,8 ;
◆ e+f inférieur ou égal à a ;
◆ a+b+2c+d+g =4 ;
◆ a+b+g compris entre 0,25 et 0,95.

Les radicaux hydrocarbonés monovalents sont e.g. des alkyles, des cycloalkyles, des aryles, des arylalkyles, des alcényles ou des alcynyles.

### La quantité de silicium de la matrice POS par rapport aux autres constituants

Le rapport atomique [(M/Si) * 100] est un autre paramètre représentatif des nanoparticules selon l'invention. Ainsi, ces nanoparticules sont-elles caractérisées par un rapport de % atomique [(M/Si) x100] compris entre 10 et 60, de préférence entre 25 et 40.

Suivant une autre caractéristique remarquable de l'invention, 1 à 80%, et de préférence 20 à 60%, des siliciums de la matrice POS des nanoparticules sont liés, par liaison silane Si-C, à au moins un chélatant C1.

En d'autres termes, 25 à 95% et de préférence de 40 à 60% des atomes de silicium de la matrice POS sont liés de façon covalente à un atome de carbone.

Avantageusement, au moins 20; 25; 30; 35; 40; 45; 50; 60%, selon un ordre croissant de préférence, des siliciums de la matrice POS des nanoparticules sont liés, par liaison silane Si-C, à au moins un groupement de fonctionnalisation chélatant C¹, et/ou à au moins un autre greffon de fonctionnalisation Gf* lié à la matrice POS par liaison covalente -Si-C-, Gf* pouvant être issu :
∘ d'un composé hydrophile (PEG) ;
∘ d'un composé comportant un principe actif PA1 ;
∘ d'un composé ciblant ;
∘ d'un composé luminescent (fluorescéine).

Dans un mode préféré de réalisation où chacune des nanoparticules comprend au moins une couche externe comprenant un greffon de fonctionnalisation chélatant C¹ greffé à la surface de la matrice POS; il est prévu qu'entre 1 et 80%, idéalement 20 et 60% des atomes de silicium, sont liés par des liaisons Si-C au greffon de fonctionnalisation chélatant C¹.

### Multimodalité

Avantageusement, ces nanoparticules peuvent être élaborées pour présenter une multimodalité de propriétés, c'est-à-dire qu'elles sont multimodales, notamment du fait des différents cations Mⁿ⁺, voire D^{m+}, qu'elles comprennent, qui sont complexés par C¹, et qui sont utiles comme agent(s) de contraste en imagerie et/ou comme agent(s) thérapeutique(s).Par exemple, des mêmes nanoparticules présentent :
→ un comportement d'agent de contraste pour au moins deux des techniques de détection, par exemple l'IRM et la scintigraphie ou l'IRM et la fluorescence,
→ ou deux comportements différents d'agent thérapeutique,
→ ou au moins un comportement d'agent de contraste et au moins un comportement d'agent thérapeutique.

Ces nouvelles nanoparticules peuvent donc être utilisées comme une plateforme permettant de combiner de nombreuses fonctionnalités (en plus de celles en IRM) : fonctions d'imagerie (fluorescence, scintigraphie...) et/ou fonctions thérapeutiques (radiosensibilisation, curiethérapie, photothérapie dynamique...).

### Relaxivité

De préférence, les nanoparticules selon l'invention ont une relaxivité r₁ par ion Mⁿ⁺ est supérieure à 5 mM⁻¹ (d'ion Mⁿ⁺).s⁻¹ préférentiellement 10 mM⁻¹ (d'ion Mⁿ⁺).s⁻¹ pour une fréquence de 20 MHz.

Mieux encore, ces nanoparticules ont une relaxivité r₁ par ion Mⁿ⁺ à 60 MHz qui est supérieure ou égale à la relaxivité r₁ par ion Mⁿ⁺ à 20 MHz.

La relaxivité r₁ considérée ici est une relaxivité par ion Mⁿ⁺ (par exemple gadolinium). r₁ est extrait de la formule suivante : 1/T₁ = [1/T₁]ₑₐᵤ + r₁[Mⁿ⁺]

### Eléments actifs métalliques (cations) M et dopants D

De préférence, M et D sont choisis dans les groupes d'éléments suivants : lanthanides, éléments de transition, actinides, éléments des colonnes Ib, IIa, IIIa, IIIb, Va, VIb, VIIb, VIII de la classification périodique selon "The Merck Index - Eleventh edition" ;
de préférence dans les sous-groupes comprenant :
∘ les lanthanides suivants : Gd, Dy, Eu, Tb, Nd, Yb, Er, Ho, Lu ;
∘ Ib : Cu, Ag, Au ;
∘ IIa : Ca, Mg ;
∘ IIIa: Ga, In ;
∘ IIIb : Y ;
∘ Va : Bi ;
∘ VIb : Cr, Mo ;
∘ VIIb : Mn, Tc ;
∘ VIII : Fe, Ru, Pt, Rh, Ir.

Gd, Dy conviennent bien e.g. pour des nanoparticules utiles comme agent de contraste en IRM.

Eu, Tb, Nd, Yb, Er conviennent bien e.g. pour des nanoparticules utiles comme agent de fluorescence.

Ho, Lu conviennent bien e.g. pour des nanoparticules utiles comme agent de Curiethérapie.

Lu, Yb, Gd, Ho conviennent bien e.g. pour des nanoparticules utiles comme agent de radio-sensibilisation.

### Localisation des éléments actifs métalliques (cations) M et dopants D

Selon une caractéristique avantageuse de l'invention, les cations Mn+ et/ou Dm+ sont localisés à la surface des nanoparticules.

D'où il s'ensuit que ces cations sont proches de molécules d'eau et peuvent ainsi avoir notamment un effet important de rehaussement de contraste T₁ en IRM. Cette amélioration des performances des nanoparticules suivant l'invention, est un témoin, parmi d'autres, de la localisation des cations Mⁿ⁺ et/ou D^{m+} à la surface.

### Chélatants C1, C2 ou C3

Avantageusement, le chélatant C1, C2 est choisi parmi les produits suivants :
∘ les produits du groupe des acides polycarboxyliques polyaminés et leurs dérivés, et, plus préférentiellement encore dans le sous-groupe comprenant: DOTA, DTPA, EDTA, EGTA, BAPTA, NOTA et leurs mélanges ;
∘ les produits du groupe comprenant porphyrine, chlorine, 1,10-phénanthroline, bipyridine, terpyridine, cyclam, triazacyclononane, leurs dérivés et leurs mélanges;
∘ et leurs mélanges.

Comme exemples de chélatants, on peut citer ceux comprenant un motif DTPA, DOTA, DTDTPA (DTPA dithiolé) ou un motif acide succinique.

Si M est un lanthanide, C1, C2 est avantageusement sélectionné parmi ceux qui présentent des propriétés complexantes des lanthanides, en particulier ceux dont la constante de complexation log(K_{C1}) est supérieure à 15, préférentiellement 20. (e.g. le DTPA ou le DOTA).

### Composés hydrophiles

Selon une disposition privilégiée de l'invention, les composés hydrophiles desquels sont issus les greffons de fonctionnalisation sont choisis dans le groupe des polyols, de préférence dans le sous-groupe comprenant les glycols, les sucres et leurs mélanges; les dextranes, le PEG et le PPG étant particulièrement préférés.

Suivant une alternative, ces composés hydrophiles peuvent être choisis parmi ceux qui ont des masses molaires inférieures à 2.000 g/mol, de préférence inférieures à 800 g/mol. On donne ci-après des exemples de composés hydrophiles, avec leur masse molaire (Mw) préférée :
- Poly(éthylèneglycol)bis(carboxyméthyl)éther (PEG), 250< Mw<2000 g.mol⁻¹ ;
- Polyoxyéthylène bis(amine), 250< Mw<2000 g.mol⁻¹ ;
- O-Méthyl-O'-succinylpolyéthylèneglycol, Mw de l'ordre de 2000 g.mol⁻¹ ;
- Méthoxypolyéthylèneglycolamine Mw de l'ordre de 750 g.mol⁻¹ ;
- Acide succinique et mercaptosuccinique ;
- Sucres, en particulier le glucose et ses dérivés, par exemple les dextranes ;
- Acides aminés ou peptides hydrophiles (acide aspartique, acide glutamique, lysine, cystéine, sérine, thréonine, glycine...) ;
- Et leurs mélanges.

Plus généralement, les composés hydrophiles comprennent avantageusement des fonctions alcools ou acides carboxyliques ou amines ou amides ou esters ou éther-oxydes ou sulfonates ou phosphonates ou phosphinates et seront liées, de préférence de façon covalente, à au moins 10 % des atomes de silicium du POS de la matrice.

Il va de soi que le chélatant C1, C2 peut être un composé hydrophile et réciproquement.

### Le principe actif PA1 ou PA2 différent de [Mⁿ⁺]/[C1] et [D^{m+}]/[C1].

Selon une modalité intéressante de l'invention, les nanoparticules portent un principe actif PA2, par l'intermédiaire de liaisons chimiques labiles, de préférence choisies dans le groupe des liaisons chimiques labiles comprenant : amide, -S-S-, ou une liaison susceptible d'être lysée par une enzyme spécifique.

Selon une autre modalité intéressante de l'invention, Le principe actif PA1 ou PA2 (e.g. inclus dans un greffon de fonctionnalisation ou complexé par C1) est choisi dans le groupe des molécules thérapeutiques, des composés magnétiques, des composés radioactifs ou susceptibles de le devenir après activation.

### Composés ciblants biologiques

Le choix des cibleurs biologiques se fait parmi les familles de biovecteurs suivantes, utilisées en imagerie et/ou en thérapie, de préférence dans le groupe des espèces capables de reconnaître et de s'apparier spécifiquement avec des zones d'intérêts biologiques et permettant ainsi un ciblage desdites zones. Il peut s'agir e.g.de : protéines, glycoprotéines, lipoprotéines, polypeptides, peptides (e.g. RGD), peptidomimétiques, colorants, sucres, oligosaccharides, neuromédiateurs, amines quaternaires, aptamères, anticorps et les associations desdits composés.

### Composés luminescents

Les composés luminescents sont choisis dans le groupe des fluorophores organiques ou inorganiques

Il va de soi que chaque produit :
∘ chélatant C1, C2 ;
∘ composé hydrophile ;
∘ principe actif PA1 ou PA2 ;
∘ composé ciblant biologique ;
∘ ou composé luminescent ;
peut posséder la caractéristique de tout ou partie des autres produits.

### Procédé

L'invention concerne aussi un procédé d'obtention de nanoparticules notamment du type de celles définies ci-dessus. Ce procédé est caractérisé en ce qu'il comprend les étapes (a), (b), (c), (d), (e) ((f) : facultative) définies ci-avant ; les étapes (c), (d), (e), (f) pouvant être réalisées dans un ordre différent ou en même temps.

L'étape (a) consiste plus précisément en pratique à former une nanoparticule de type coeur/coquille avec un coeur d'oxyde de lanthanide (par voie polyol modifiée) et une coquille de polysiloxane (par sol/gel), cet objet a par exemple une taille aux alentours de 10 nm (préférentiellement 5 nanomètres). Un coeur d'oxyde de lanthanide de taille très petite (adaptable inférieure à 10 nm) peut ainsi être élaboré dans un alcool par un des procédés décrits dans les publications suivantes (P. Perriat et al., J. Coll. Int. Sci, 2004, 273, 191 ; O. Tillement et al., J. Am. Chem. Soc., 2007, 129, 5076 et P. Perriat et al., J. Phys. Chem. C, 2009, 113, 4038).

Selon l'étape (b), ces coeurs peuvent être enrobés par une couche de polysiloxane en suivant par exemple un protocole décrit dans les publications suivantes (C. Louis et al., Chem. Mat., 2005, 17, 1673 et O. Tillement et al., J. Am. Chem. Soc., 2007, 129, 5076).

Dans l'étape (c), on greffe à la surface du polysiloxane des chélatants spécifiques des cations métalliques visés ; on peut également en insérer une partie à l'intérieur de la couche mais le contrôle de la formation du polysiloxane est complexe et le simple greffage extérieur donne, à ces très faibles tailles, une proportion de greffage suffisante.

L'étape (d) de purification consiste plus précisément à séparer les nanoparticules des résidus de synthèse par une méthode de dialyse ou de filtration tangentielle, sur une membrane comportant des pores de taille adaptée.

Dans l'étape (e) ultérieure, le coeur est détruit par dissolution (par exemple en modifiant le pH ou en apportant des molécules complexantes dans la solution). Cette destruction du coeur permet alors un éparpillement de la couche de polysiloxane (selon un mécanisme d'effondrement ou de corrosion lente), ce qui permet d'obtenir en final un objet en polysiloxane de morphologie complexe dont les dimensions caractéristiques sont de l'ordre de grandeur de l'épaisseur de la couche de polysiloxane, c'est-à-dire beaucoup plus petit que les objets jusqu'à présent élaborés. On obtient également une forte teneur en chélatants C1 puisque ceux-ci sont greffés initialement à la surface du polysiloxane et qu'à ces très faibles tailles la surface concerne une très forte proportion de la matière de la particule, le rapport surface sur volume varie en fonction de la taille en 1/r (rayon). Au cours du mécanisme d'effondrement de cette structure d'autres complexes peuvent se fixer également jusqu'à saturation sur les surfaces « fraîches » nouvellement formées. On atteint ainsi des teneurs en complexants largement supérieures à celles qui auraient été obtenues par une classique fonctionnalisation de surface de particules de silice plus fines, sous réserve de la disponibilité de telles particules.

Le fait de retirer le coeur permet ainsi de passer d'une taille de particules d'environ 5 nanomètres de diamètre à une taille d'environ 3 nanomètres. De plus, cette opération permet d'augmenter le nombre de M (e.g. gadolinium) par nm³ en comparaison d'une nanoparticule de polysiloxane théorique de même taille mais comprenant du M (e.g. gadolinium) uniquement en surface.

Le nombre de M pour une taille de nanoparticule est évaluable grâce au rapport atomique M/Si mesuré par EDX.

### Produits dérivés des nanoparticules

L'invention a également pour objet :
- Une suspension de nanoparticules telles que définies ci-dessus et/ou obtenues par le procédé décrit supra.
- Une suspension de nanoparticules telles que définies ci-dessus et/ou obtenues par le procédé décrit supra, caractérisée en ce qu'elle comprend du chélatant C¹ libre d'ions Mⁿ⁺ ou D^{m+}.
- Une matière solide obtenue par élimination du liquide, de préférence par lyophilisation de la suspension de nanoparticules telles que définies ci-dessus et/ou obtenues par le procédé décrit supra.
- Un liquide injectable comprenant des nanoparticules telles que définies ci-dessus et/ou obtenues par le procédé décrit supra, et/ou de la suspension de ces nanoparticules et/ou préparé à partir de la matière solide susvisée.

### Applications

L'invention se rapporte également aux applications de ces nanoparticules, dont notamment :
- A une composition d'aide au diagnostic, de préférence composition de contraste, caractérisée en ce qu'elle comprend des nanoparticules telles que définies ci-dessus et/ou obtenues par le procédé décrit supra et/ou de la suspension susvisée et/ou préparé à partir de la matière solide définie ci-dessus.
- A une composition thérapeutique caractérisée en ce qu'elle comprend des nanoparticules telles que définies ci-dessus et/ou obtenues par le procédé décrit supra et/ou de la suspension susvisée et/ou préparé à partir de la matière solide définie ci-dessus.
- A une composition thérapeutique comprenant des nanoparticules telles que définies ci-dessus et/ou obtenues par le procédé décrit supra et/ou de la suspension susvisée et/ou préparée à partir de la matière solide définie ci-dessus, pour le traitement des cancers ou des maladies neuro-dégénératives, en particulier pour le ciblage de sites à traiter.
- A une composition de marquage cellulaire caractérisée en ce qu'elle comprend des nanoparticules telles que définies ci-dessus et/ou obtenues par le procédé décrit supra et/ou de la suspension susvisée et/ou préparé à partir de la matière solide définie ci-dessus.

### Modes de réalisation

**1.** Nanoparticules caractérisées
   (i) en ce qu'elles comprennent chacune
      - une matrice polyorganosiloxane (POS) incluant des cations Mⁿ⁺ (n=2 à 6) de préférence de terre rare, éventuellement en partie sous forme d'un oxyde et/ou d'un oxohydroxyde métallique (M), éventuellement associé à des cations dopants D^{m+} (m=2 à 6), de préférence une terre rare différente de M, un actinide et/ou un élément de transition ;
      - un greffon de fonctionnalisation C¹ chélatant qui est :
         * issu d'un chélatant C1,
         * lié à la matrice POS par liaison covalente -Si-C-,
         * et en quantité suffisante pour pouvoir complexer tous les cations Mⁿ⁺ et D^{m+} ; le greffon C¹ étant de préférence en excès par rapport aux cations Mⁿ⁺ et D^{m+} ; le chélatant C1 duquel le greffon C¹ est issu correspondant à une ou plusieurs espèces différentes ;
      - éventuellement un autre greffon de fonctionnalisation Gf* lié à la matrice POS par liaison covalente -Si-C-, Gf* pouvant être issu :
         * d'un composé hydrophile,
         * d'un composé comportant un principe actif PA1,
         * d'un composé ciblant,
         * d'un composé luminescent ;
   (ii) en ce que leur diamètre d₁ est compris entre 1 et 20 nm, de préférence entre 1 et 10 nm ;
   (iii) et éventuellement en ce qu'elles sont porteuses d'un principe actif PA2, identique ou différent à PA1.
**2.** Nanoparticules en particulier selon le mode 1 caractérisées en ce que la matrice POS de chaque nanoparticule est obtenue à partir d'une nanoparticule de diamètre d₀ comprenant:
   - un coeur à base d'un oxyde et/ou un oxohydroxyde métallique (M), de préférence de terre rare, au moins en partie sous forme cationique Mⁿ⁺ (n=2 à 6), éventuellement dopé par un dopant (D), de préférence une terre rare différente de M, un actinide et/ou un élément de transition ;
   - au moins une couche d'enrobage à base de POS ;
   - et, éventuellement un surenrobage à base d'un agent de fonctionnalisation, de préférence choisi parmi les chélatants de fonctionnalisation C1 aptes à séquestrer des cations et/ou les composés ciblants et/ou les composés comportant un principe actif PA1 et/ou les composés hydrophiles et/ou les composés luminescents ;
   ladite nanoparticule étant soumise à une dissolution du coeur M, de préférence à l'aide d'un agent modificateur du pH et/ou d'un chélatant C2, identique ou différent à C1, apte à complexer tout ou partie des cations Mⁿ⁺ et D^{m+}, de sorte que le diamètre d₀ de la nanoparticule est réduit à une valeur d₁ comprise entre 1 et 20 nm, de préférence entre 1 et 10 nm.
**3.** Nanoparticules selon au moins un des modes précédents caractérisées par leur masse molaire (en kDa) supérieure ou égale à, dans un ordre croissant de préférence, 2,3,5, et inférieure ou égale à, dans un ordre croissant de préférence, 200,100,50,20,10.
**4.** Nanoparticules selon au moins un des modes précédents caractérisées par la formule générale suivante :

   **[C¹]ₐ[R]_{b}Si[O]_{c}[OH]_{d}[Mⁿ⁺]ₑ[D^{m+}]_{f}[Gf*]_{g}**

   avec :
   ◆ C¹ sont des greffons de fonctionnalisation formés par des radicaux hydrocarbonés monovalents, identiques ou différents entre eux, chélatants et reliés chacun au Si par une liaison Si-C ;
   ◆ R sont des radicaux monovalents, identiques ou différents entre eux, constituant un greffon de fonctionnalisation relié au Si par une liaison Si-C, et comprenant de préférence un groupe hydrophile et au moins un atome N ou O ;
   ◆ Mⁿ⁺ sont des cations métalliques identiques ou différents entre eux avec n=2 à 6;
   ◆ D^{m+} sont des cations métalliques identiques ou différents entre eux avec m=2 à 6 ;
   ◆ Gf* sont des greffons de fonctionnalisation autres que C¹ et formés par des radicaux hydrocarbonés monovalents, identiques ou différents entre eux, reliés chacun au Si par une liaison Si-C et pouvant être issu :
      ∘ d'un composé hydrophile ;
      ∘ d'un composé comportant un principe actif PA1 ;
      ∘ d'un composé ciblant ;
      ∘ d'un composé luminescent ;
   ◆ a supérieur ou égal à 0,01 et inférieur ou égal à 0,8 ; avec de préférence a ≥ d + e;
   ◆ b inférieur ou égal à 0,7 ;
   ◆ c supérieur ou égal à 0,5 et inférieur à 1,9 ;
   ◆ g supérieur ou égal à 0 et inférieur à 0,3 ;
   ◆ e+f supérieur ou égal à 0,01 et inférieur ou égal à 0,8 ;
   ◆ e+f inférieur ou égal à a ;
   ◆ a+b+2c+d+g =4 ;
   ◆ a+b+g compris entre 0,25 et 0,95.
**5.** Nanoparticules selon au moins un des modes précédents caractérisés en ce qu'au moins 1% et de préférence au moins 10% des chélatants C¹ ne sont pas complexés par des cations Mⁿ⁺ et/ou D^{m+}.
**6.** Nanoparticules selon au moins un des modes précédents caractérisés en ce que M et /ou D est un agent actif en imagerie et/ou en thérapie.
**7.** Nanoparticules selon au moins un des modes précédents caractérisées en ce que les cations Mⁿ⁺ et/ou D^{m+} sont localisés à la surface des nanoparticules.
**8.** Nanoparticules selon au moins un des modes précédents caractérisées en ce qu'elles ne comprennent pas de coeur cristallisé.
**9.** Nanoparticules selon au moins un des modes précédents caractérisées par un rapport de % atomique [(M/Si) x100] compris entre 10 et 60, de préférence entre 25 et 40.
**10.** Nanoparticules selon au moins un des modes précédents caractérisées en ce que la relaxivité r₁ par ion Mⁿ⁺ est supérieure à 5 mM⁻¹ (d'ion Mⁿ⁺).s⁻¹ préférentiellement 10 mM⁻¹(d'ion Mⁿ⁺).s⁻¹ pour une fréquence de 20 MHz.
**11.** Nanoparticules selon au moins un des modes précédents caractérisées en ce que la relaxivité r₁ par ion Mⁿ⁺ à 60 MHz est supérieure à la relaxivité r₁ par ion Mⁿ⁺ à 20 MHz.
**12.** Procédé d'obtention des nanoparticules selon au moins un des modes précédents caractérisé en ce qu'il comprend les étapes suivantes :
   a Synthèse de coeurs à base d'un oxyde et/ou un oxohydroxyde métallique (M), de préférence de terre rare, au moins en partie sous forme cationique Mⁿ⁺ (n=2 à 6), éventuellement dopé par un dopant (D), de préférence une terre rare différente de M, un actinide et/ou un élément de transition ;
      cette synthèse consistant essentiellement à mélanger une base avec un sel de M dissous dans un solvant de préférence choisi dans le groupe comprenant les alcools ;
   b Enrobage des coeurs de l'étape (a) par du polyorganosiloxane (POS) consistant essentiellement à mettre en oeuvre une technique sol/gel d'hydrolyse-condensation d'espèces siliciques et d'alcoxysilanes, en présence d'un base ou d'un acide et éventuellement d'un principe actif PA1 et/ou PA2 ;
   c Surenrobage de fonctionnalisation des coeurs enrobés de l'étape (b) consistant essentiellement à mettre en présence ces coeurs enrobés de l'étape (b) en présence d'un précurseur de greffons de fonctionnalisation C¹, et éventuellement en présence d'un greffon de fonctionnalisation Gf* ;
   d Purification des nanoparticules surenrobées/fonctionnalisées de diamètre d₀, de préférence par filtration tangentielle, dialyse et/ou par précipitation/ lavage ;
   e Dissolution des coeurs M des nanoparticules surenrobées/fonctionnalisées de l'étape (c) consistant essentiellement à les mettre en présence d'un agent modificateur du pH et/ou d'un chélatant C2 apte à complexer tout ou partie des cations Mⁿ⁺ et D^{m+}, de sorte que le diamètre d₀ des nanoparticules est réduit à une valeur d₁ comprise entre 1 et 20 nm, de préférence entre 1 et 10 nm ;
   f Eventuel ajout d'un sel cationique destiné à être au moins en partie complexé par le chélatant C¹ ;
   les étapes (c), (d), (e), (f) pouvant être réalisées dans un ordre différent ou en même temps.
**13.** Suspension de nanoparticules selon au moins un des modes 1 à 10 et/ou obtenues par le procédé selon le mode de réalisation 12.
**14.** Matière solide obtenue par élimination du liquide, de préférence par lyophilisation de la suspension selon le mode de réalisation 13.
**15.** Liquide injectable comprenant des nanoparticules selon au moins un des modes 1 à 11 et/ou obtenues par le procédé selon la revendication 12 et/ou de la suspension selon la revendication 13 et/ou préparé à partir de la matière solide selon la revendication 14.

### EXEMPLES

### Matériel et méthodes

- La mesure de la taille des nanoparticules en suspension colloïdale s'effectue par spectroscopie de corrélation de photons (PCS) sur un Zetasizer Nano-S de Malvern.
- La taille des particules peut également être mesurée par microscopie électronique en transmission (TEM), sur un microscope JEOL 2010 avec une tension d'accélération de 200 kV. Une goutte de solution colloïdale diluée est placée sur une grille de carbone pour l'analyse.
- La centrifugation des solutions s'effectue à l'aide d'une centrifugeuse Allegra 25R de Beckman Coulter, à une vitesse de 4100 rpm.
- Dans le cadre de la purification, la filtration tangentielle des solutions s'effectue à travers une membrane de 5kDa, dans des tubes de Vivaspin 20mL de Sartorius Stedim Biotech, jusqu'à obtenir une dilution des impuretés supérieure à 1000. Pour cela les tubes de Vivaspin sont centrifugés à 4100 rpm également.
- La lyophilisation des particules s'effectue sous vide dynamique à 0,15 mbar et -2°C dans un lyophilisateur CHRIST Alpha 1-2.
- Les analyses chimiques par diffraction de rayons X (EDX) s'effectuent sur un microscope électronique à balayage Philips XL20. Pour chaque échantillon, une goutte de solution aqueuse est déposée sur un support en carbone. Après séchage, la surface est métallisée par une couche d'or de 10 nm d'épaisseur. Pour l'analyse chimique trois mesures sont moyennées, sur trois zones de 50µm x 50µm environ. Les pourcentages de chaque élément sont obtenus en pourcentages atomiques.
- Le signal des particules en relaxométrie est mesuré à l'aide d'un analyseur Bruker Minispec MQ60 NMR, avec un champ magnétique de 1,4T.

### Exemple 1. Synthèse de coeur d'oxyde de gadolinium

Une solution est préparée par dissolution d'une quantité de 11 g/L de sel de chlorure de gadolinium (GdCl₃, 6H₂O) dans un volume de 375 mL de diéthylène glycol (DEG). A la solution obtenue, un ajout de 375 mL d'une solution de soude à 0,1 mol/L dans du DEG est effectué, à température ambiante, en 15h.

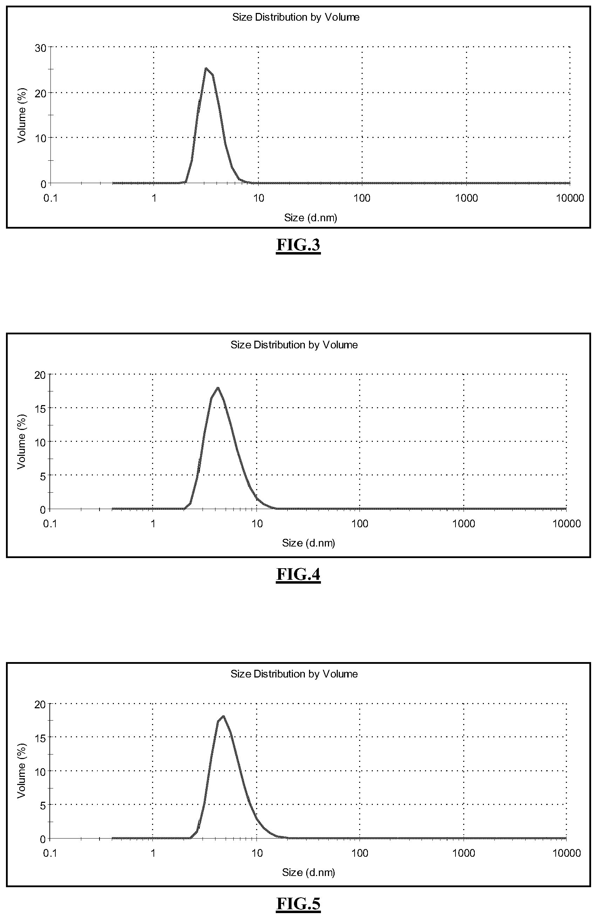

La figure 3 annexée montre la distribution de taille des coeurs d'oxyde de gadolinium, mesurée dans le DEG par PCS ; moyenne : 3,6 nm.

### Exemple 2. Fonctionnalisation des coeurs d'oxyde de gadolinium avec du DTPABA

Autour des coeurs d'oxyde de gadolinium de l'exemple 1, une couche de polysiloxane fonctionnalisé est synthétisée par voie sol-gel. Pour ce faire, la solution de coeurs de 750 mL est portée à 40°C dans un bain d'huile, sous agitation. 787 µL d'APTES, 502 µL de TEOS et 1913 µL d'une solution aqueuse de triéthylamine à 0,1 mol/L sont ajoutés à la solution de coeurs. Ces ajouts sont répétés une deuxième fois après 24h d'attente. La solution est laissée ensuite 48h à 40°C sous agitation. On obtient des particules coeur-écorce d'une taille de 5 nm environ, avec des fonctions amines en surface. La figure 4 annexée montre la distribution de taille des coeurs enrobés polysiloxane, mesurée dans le DEG par PCS ; moyenne : 4,9 nm.

Ensuite 3,135g de DTPABA sont dispersés dans 150 mL de diméthyl sulfoxide (DMSO). Puis les 750 mL de solution de coeurs d'oxyde de gadolinium sont ajoutés à la solution de DTPABA. Le mélange est agité pendant 24h.

Les nanoparticules sont ensuite précipitées par centrifugation dans quatre bidons de 500 mL d'acétone. Puis l'acétone est retirée, et les particules sont redispersées dans 100 mL d'eau au total. Elles sont ensuite purifiées par filtration tangentielle. Les impuretés de grande taille sont retirées par filtration à l'aide d'une seringue, à travers une membrane de 0,2 µm.

On obtient des particules d'une taille de 5,5 nm environ. La figure 5 annexée montre la distribution de taille des coeurs enrobés polysiloxane, fonctionnalisés DTPABA, mesurée dans l'eau par PCS ; moyenne : 5,6 nm.
La figure 6 annexée montre le suivi de la synthèse en granulométrie : en trait mixte (----) la distribution de taille des coeurs seuls ; en trait plein épais ( 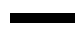 ) la distribution de taille des coeurs enrobés polysiloxane ; en trait plein fin (□) la distribution de taille des coeurs enrobés polysiloxane et fonctionnalisés DTPABA.
La figure 7 annexée montre l'image en TEM des particules fonctionnalisées DTPABA ; les tailles observées sont comprises entre 4 et 6 nm.

La solution aqueuse de nanoparticules peut être conservée plusieurs mois au réfrigérateur après lyophilisation, dans des piluliers scellés hermétiquement.

L'analyse chimique réalisée par Spectroscopie de masse - plasma couplé par induction (ICP-MS) au Service Central d'Analyse de Solaize, donne les résultats suivants en pourcentages atomiques : carbone 70,6%, azote 14,3%, gadolinium 4,9%,

### Exemple 3. Dissolution du coeur des particules d'oxyde de gadolinium fonctionnalisées DTPABA à l'aide d'acide chlorhydrique

Aux particules de l'exemple 2, dispersées dans l'eau, on ajoute de l'acide chlorhydrique concentré jusqu'à l'obtention d'un pH égal à 2,5. On laisse agiter une nuit.

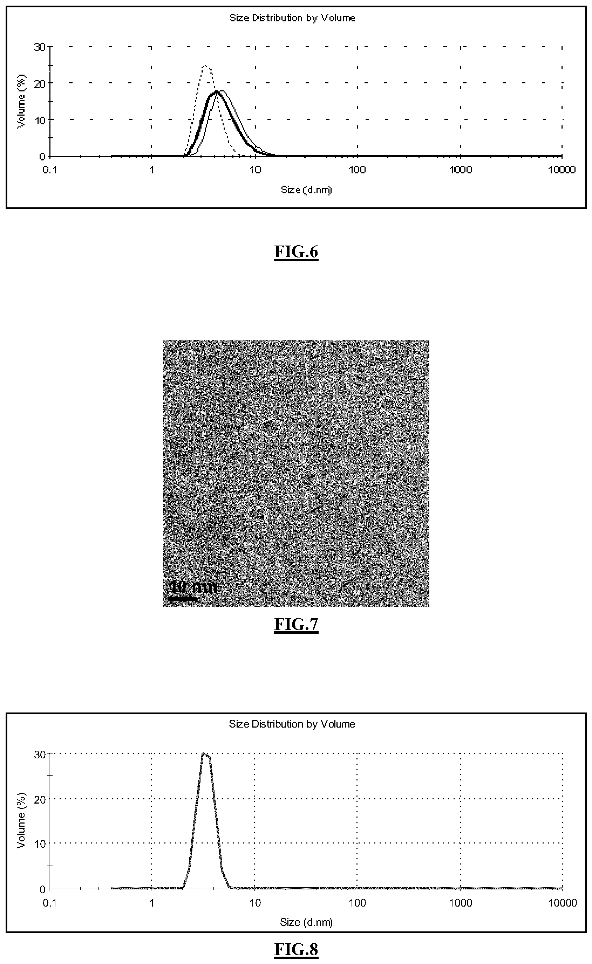

La solution est purifiée par filtration tangentielle, pour éliminer les ions Gd³⁺ qui ont été dissous à partir du coeur des particules. On obtient des particules d'une taille de 3,5 nm environ. La figure 8 annexée montre la distribution de taille des particules diminuées à l'acide chlorhydrique, mesurée dans l'eau par PCS ; moyenne : 3,4 nm.

Le signal des particules en relaxométrie r₁=1/T₁ a diminué de 27%. Une importante attaque chimique a été observée avec dissolution forcée par l'attaque acide. La forte diminution de la relaxométrie le confirme.

D'autre part, l'analyse chimique réalisée par EDX donne un rapport atomique gadolinium/silicium de 22,1%. Par rapport aux particules de l'exemple 2, on observe que 54% du gadolinium a été dissous par l'attaque acide.

### Exemple 4. Dissolution du coeur des particules d'oxyde de gadolinium fonctionnalisées DTPABA à l'aide de DTPA libre

Aux particules de l'exemple 2, dispersées dans l'eau, on ajoute du DTPA libre en large excès. On ajuste le pH de la solution à 7. On laisse agiter une nuit.

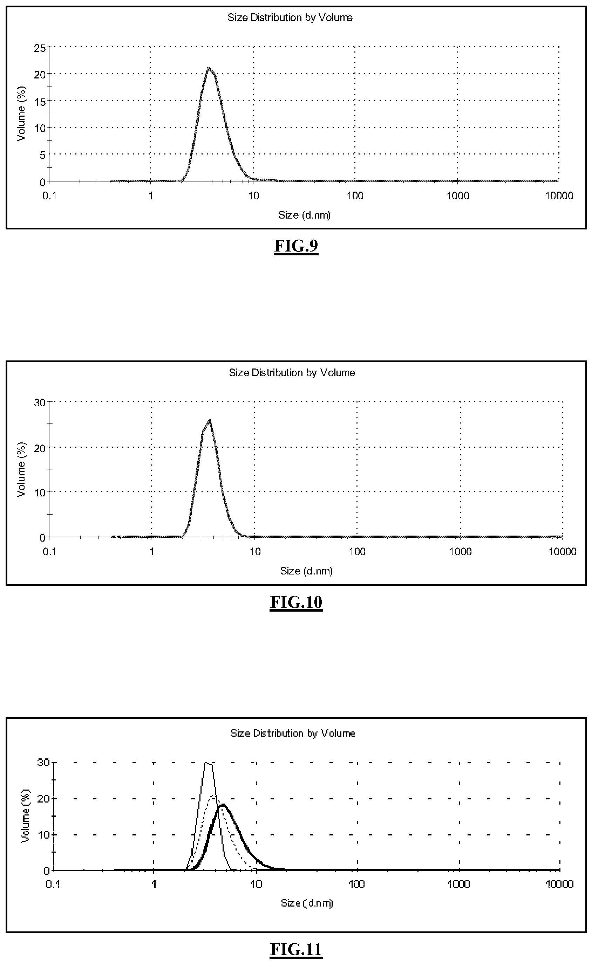

La solution est purifiée par filtration tangentielle, pour éliminer les ions Gd³⁺ qui ont été dissous à partir du coeur des particules, et complexés par le DTPA libre. On obtient des particules d'une taille de 4 nm environ. La figure 9 annexée montre la distribution de taille des particules diminuées avec ajout de DTPA, mesurée dans l'eau par PCS ; moyenne : 4,3 nm.

De plus le signal des particules en relaxométrie r₁ a diminué de 71% par rapport aux particules de l'exemple 2. Nous observons une attaque chimique très importante du coeur par dissolution forcée par la complexation. Une perte des Gd³⁺ complexés sur les DTPA greffés en surface est aussi probable, ce qui explique la plus forte diminution du signal en relaxométrie par rapport à l'exemple 3.

D'autre part, l'analyse chimique réalisée par EDX donne un rapport atomique gadolinium/silicium de 10,8%. Par rapport aux particules de l'exemple 2, on observe que 78% du gadolinium a donc été éliminé par la complexation du DTPA.

Pour évaluer la perte des Gd³⁺ complexés sur les DTPA greffés en surface, il a été choisi d'ajouter de nouveaux ions Gd³⁺ pour remplir à nouveau ces complexes. On dissout dans de l'eau une quantité d'ions Gd³⁺ équivalente à ce qu'on avait perdu dans l'étape précédente. Le pH de cette solution est ramené à 6,5. Puis on ajoute cette solution aux nanoparticules de taille diminuée. On laisse agiter une nuit.

La solution est purifiée par filtration tangentielle, pour éliminer les ions Gd³⁺ qui n'ont pas été complexés par le DTPA. On obtient des particules d'une taille de 3,5 nm environ. La figure 10 annexée montre la distribution de taille des particules diminuées avec ajout de DTPA, puis avec ajout de Gd3+, mesurée dans l'eau par PCS ; moyenne : 3,7 nm.

De plus le signal des particules en relaxométrie r1 a augmenté de 123% par rapport aux particules de taille diminuée. Nous observons donc un remplissage des DTPA restés vides avec les ions Gd³⁺ apportés à la solution. La taille des particules reste quasiment inchangée.

La figure 11 résume les expériences de diminution de tailles précédentes : en trait plein épais ( 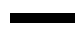 ) la distribution de taille des particules initiales ; en trait plein fin (□) la distribution de taille des particules diminuées avec HCl ; en trait mixte (----) la distribution de taille des particules diminuées avec le DTPA libre.

### Exemple 5. Dissolution des coeurs d'oxyde de gadolinium enrobés polysiloxane, directement à l'aide d'un ajout de DTPABA en large excès

Des particules sont synthétisées selon le protocole décrit dans l'exemple 2, en faisant toutefois varier la quantité de DTPABA ajoutée pour fonctionnalisation. Cette quantité varie d'un ratio DTPABA/APTES de 0,5 à 3. Le nombre d'APTES est choisi comme paramètre car il représente le nombre de fonctions potentiellement réactives à la surface des particules.

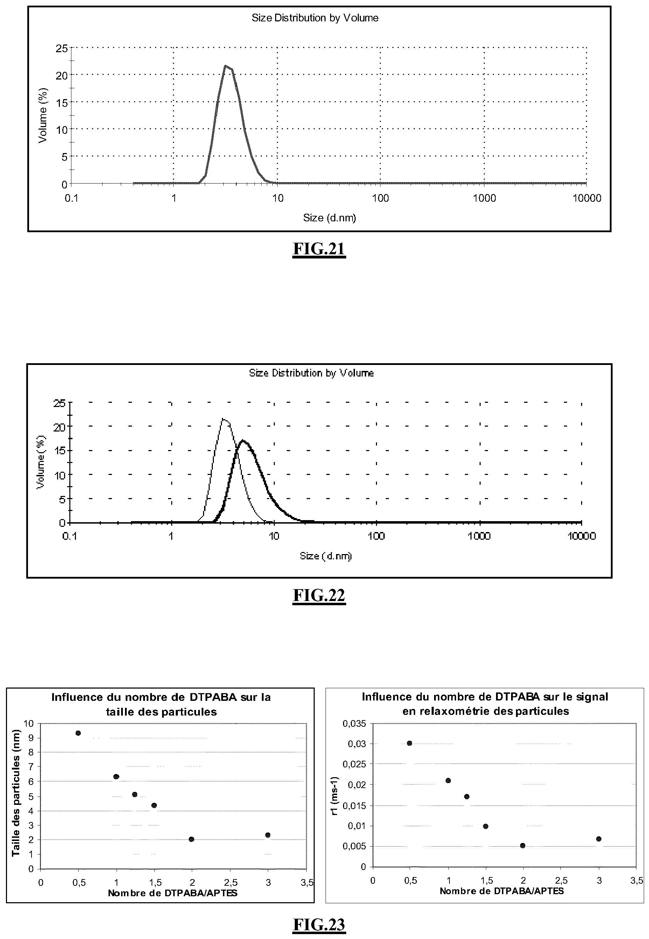

La figure 23 annexée montre la variation de la taille (à gauche) et du signal de relaxométrie (à droite), avec le nombre de DTPABA ajouté pour fonctionnalisation. Le signal en relaxométrie est mesuré en solution aqueuse diluée par 10. Pour les quantités de DTPABA faibles, les nanoparticules conservent une grande taille. Pour les quantités de DTPABA élevées, en revanche, la taille des particules vue en PCS diminue, car le coeur des particules se dissout. Pour les valeurs de 2 et 3 DTPABA/APTES, le coeur des particules est entièrement détruit, et la taille des particules stagne à 2 nm environ. Le signal en relaxométrie des particules diminue proportionnellement à la taille, ce qui traduit la diminution proportionnelle de la quantité de gadolinium présent dans les coeurs.

### Exemple 6. Fonctionnalisation des coeurs d'oxyde de gadolinium avec DOTA-NHS et purification

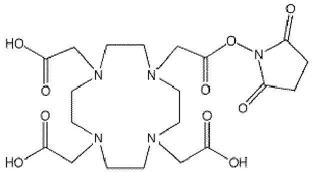

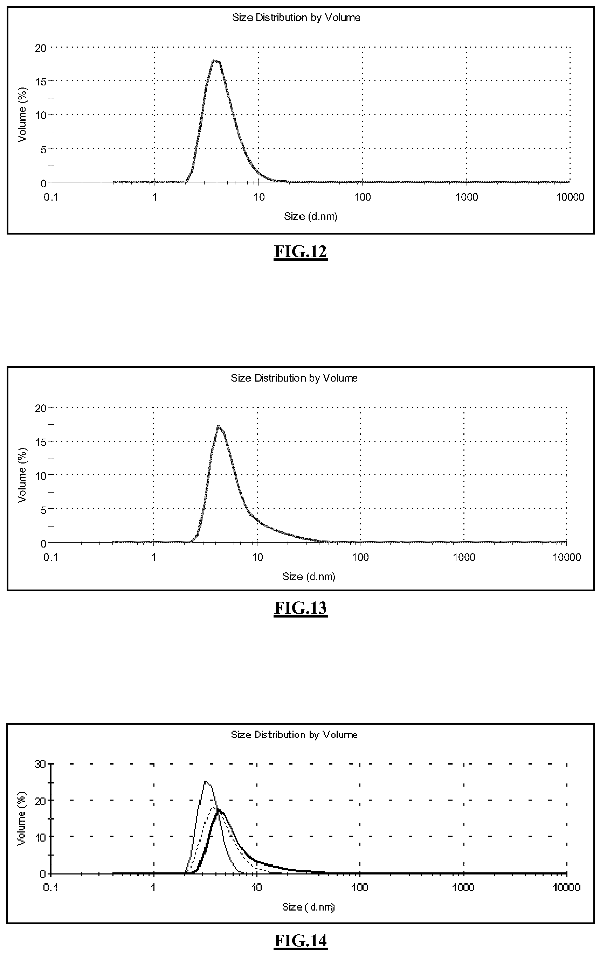

Autour des coeurs d'oxyde de gadolinium de l'exemple 1, une couche de polysiloxane fonctionnalisé est synthétisée par voie sol-gel. Pour ce faire, la solution de coeurs de 750 mL est portée à 40°C dans un bain d'huile, sous agitation. 787 µL d'APTES couplé à 0,75 mg de fluorescéine isothiocyanate (FITC), 502 µL de TEOS et 1913 µL d'une solution aqueuse de triéthylamine à 0,1 mol/L sont ajoutés à la solution de coeurs. Ces ajouts sont répétés une deuxième fois après 24h d'attente, et une troisième fois après 48h d'attente. La solution est laissée ensuite 48h à 40°C sous agitation. On obtient des particules coeur-écorce d'une taille de 5 nm environ, avec des fonctions amines en surface. La figure 12 annexée montre la distribution de taille des coeurs enrobés polysiloxane, mesurée dans le DEG par PCS ; moyenne : 4,7 nm.

1,12 g de DOTA-NHS sont dispersés dans 20 mL d'éthanol anhydre. Puis 100 mL de la solution de coeurs d'oxyde de gadolinium sont ajoutés à la solution de DOTA-NHS. Le mélange est agité pendant 24h.

Les nanoparticules sont ensuite précipitées dans 500 mL d'acétone. Puis l'acétone est retirée, et les particules sont redispersées dans 20 mL d'eau. Elles sont ensuite purifiées par filtration tangentielle. Les impuretés de grande taille sont retirées par filtration à l'aide d'une seringue, à travers une membrane de 0,2 µm. On obtient des particules d'une taille de 7 nm environ. La figure 13 annexée montre la distribution de taille des coeurs enrobés polysiloxane, fonctionnalisés DOTA-NHS, mesurée dans l'eau par PCS ; moyenne : 6,8 nm.

La figure 14 annexée montre le suivi de la synthèse précédente en granulométrie : en trait plein fin (□) la distribution de taille des coeurs seuls ; en trait mixte (----) la distribution de taille des coeurs enrobés polysiloxane ; en trait plein épais ( 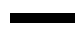 ) la distribution de taille des coeurs enrobés polysiloxane et fonctionnalisés DOTA-NHS.

### Exemple 7. Dissolution du coeur des particules d'oxyde de gadolinium fonctionnalisées DOTA-NHS à l'aide de DTPA libre

Aux particules de l'exemple 6, dispersées dans l'eau, on ajoute du DTPA libre en large excès. On ajuste le pH de la solution à 7. On laisse agiter une nuit.

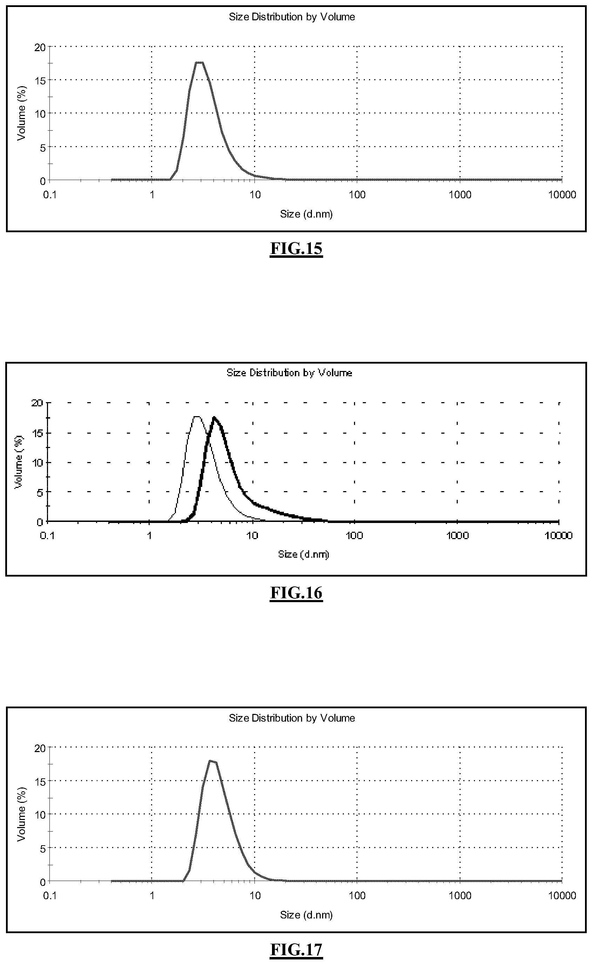

La solution est purifiée par filtration tangentielle, pour éliminer les ions Gd³⁺ qui ont été dissous à partir du coeur des particules. On obtient des particules d'une taille de 3,5 nm environ. La figure 15 annexée montre la distribution de taille des particules diminuées avec ajout de DTPA, mesurée dans l'eau par PCS ; moyenne : 3,7 nm.

De plus le signal des particules en relaxométrie r1 a diminué de 84%, ce qui traduit une forte diminution de la quantité de gadolinium présent dans les particules.

La figure 16 annexée montre l'expérience de diminution de tailles : en trait plein épais ( 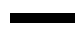 ) la distribution de taille des particules initiales ; en trait plein fin (□) la distribution de taille des particules diminuées avec le DTPA libre.

### Exemple 8. Fonctionnalisation des coeurs d'oxyde de gadolinium avec du 1,4,7,10-Tetraazacyclo dodecane-1,4,7,10-tetraacetic acid anhydre (DOTA anhydre)

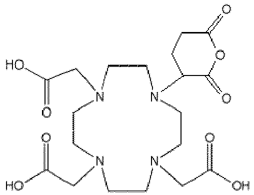

Autour des coeurs d'oxyde de gadolinium de l'exemple 1, une couche de polysiloxane fonctionnalisé est synthétisée par voie sol-gel. Pour ce faire, la solution de coeurs de 750 mL est portée à 40°C dans un bain d'huile, sous agitation. 787 µL d'APTES couplé à 0,75 mg de FITC, 502 µL de TEOS et 1913 µL d'une solution aqueuse de triéthylamine à 0,1 mol/L sont ajoutés à la solution de coeurs. Ces ajouts sont répétés une deuxième fois après 24h d'attente, et une troisième fois après 48h d'attente. La solution est laissée ensuite 48h à 40°C sous agitation. On obtient des particules coeur-écorce d'une taille de 4,5 nm environ, avec des fonctions amines en surface. La figure 17 annexée montre la distribution de taille des coeurs enrobés polysiloxane, mesurée dans le DEG par PCS ; moyenne : 4,7 nm.

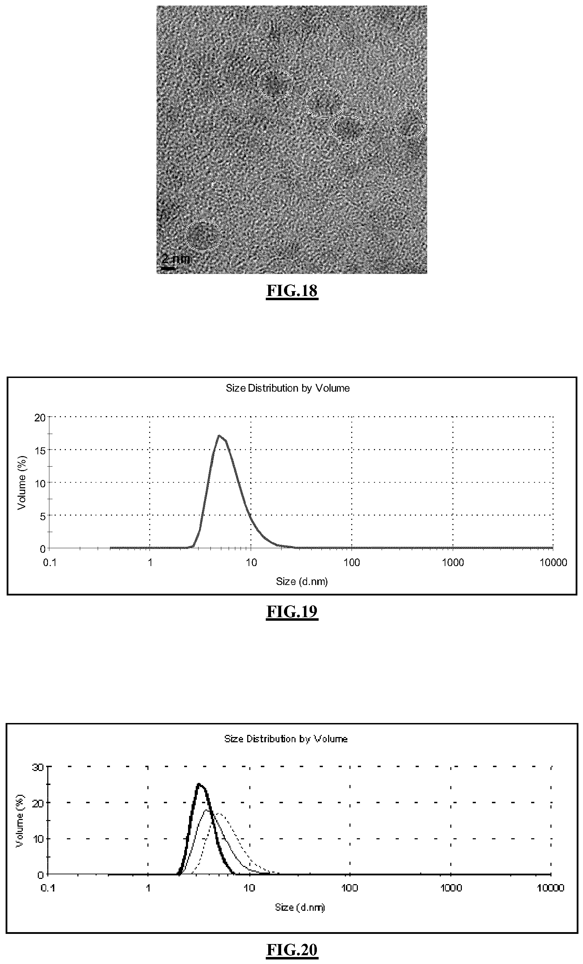

La taille des particules est aussi mesurée par TEM. La figure 18 annexée montre l'image en TEM des coeurs enrobés polysiloxane, dispersées à l'aide du greffage de polyéthylène glycol ; les tailles observées sont comprises entre 4 et 5 nm.

Ensuite 0,619 g de DOTA anhydre sont dispersés dans 20 mL de DMSO. Puis 100 mL de la solution de coeurs d'oxyde de gadolinium sont ajoutés à la solution de DOTA anhydre. Le mélange est agité pendant 24h.

### Exemple 9. Purification des particules d'oxyde de gadolinium fonctionnalisées DOTA anhydre

Les nanoparticules de l'exemple 8 sont précipitées dans 500 mL d'acétone. Puis l'acétone est retirée, et les particules sont redispersées dans 20 mL d'eau. Elles sont ensuite purifiées par filtration tangentielle. Les impuretés de grande taille sont retirées par filtration à l'aide d'une seringue, à travers une membrane de 0,2 µm. On obtient des particules d'une taille de 6,5 nm environ. La figure 19 annexée montre la distribution de taille des coeurs enrobés polysiloxane, fonctionnalisés DOTA anhydre, mesurée dans l'eau par PCS ; moyenne : 6,3 nm.

La figure 1 annexée montre l'image en TEM des coeurs enrobés polysiloxane, fonctionnalisés DOTA anhydre ; les tailles observées son comprises entre 3 et 5 nm. Les molécules de DOTA ne sont pas visibles au TEM, on ne voit que les coeurs enrobés.

La figure 20 annexée montre le suivi de la synthèse en granulométrie : en trait plein épais ( 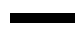 ) la distribution de taille des coeurs seuls ; en trait plein fin (□) la distribution de taille des coeurs enrobés polysiloxane ; en trait mixte (----) la distribution de taille des coeurs enrobés polysiloxane et fonctionnalisés DOTA anhydre.

L'analyse chimique réalisée par EDX donne un rapport atomique gadolinium/silicium de 57,2%.

La solution de nanoparticules peut être conservée plusieurs mois au réfrigérateur après lyophilisation.

### Exemple 10. Dissolution du coeur des particules d'oxyde de gadolinium fonctionnalisées DOTA anhydre à l'aide de DTPA libre

Une solution de DTPA dans l'eau est préparée, puis le pH de la solution est ramené à 6. Aux particules de l'exemple 8, dispersées dans le mélange de solvants DEG et DMSO, on ajoute de la solution de DTPA en large excès. On laisse agiter une nuit.

La solution est purifiée par filtration tangentielle, pour éliminer les ions Gd³⁺ qui ont été dissous à partir du coeur des particules. Par la même occasion les particules sont passées dans l'eau. On obtient des particules d'une taille de 3,5 nm environ. La figure 21 annexée montre la distribution de taille des particules diminuées avec ajout de DTPA, mesurée dans l'eau par PCS ; moyenne : 3,6 nm.

La figure 2 annexée montre l'image en TEM des particules dont on a diminué la taille. L'image a été prise dans les mêmes conditions que les précédentes. De plus l'analyse chimique locale indique la présence de Gadolinium en quantité significative à l'endroit observé, mais aucune particule n'y est visible. Les particules n'ont donc plus de coeur cristallisé à ce stade.

La figure 22 annexée montre l'expérience de diminution de tailles : en trait plein épais ( 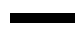 ) la distribution de taille des particules initiales ; en trait plein fin (□) la distribution de taille des particules diminuées avec le DTPA libre.

De plus le signal des particules en relaxométrie r1 a diminué de 71%. Ceci traduit une forte diminution de la quantité de gadolinium présent dans les particules.

D'autre part, l'analyse chimique réalisée par EDX donne un rapport atomique gadolinium/silicium de 24,1%. Par rapport aux particules de l'exemple 9, on observe que 58% du gadolinium a donc été dissous par la complexation du DTPA.

### Exemple 11. Synthèse directe de nanoparticules fonctionnalisées DOTA anhydre, sans coeur

Des nanoparticules sont synthétisées selon le protocole décrit dans l'exemple 8, mais la fonctionnalisation de surface se fait avec un large excès de DOTA anhydre (2 DOTA/Gd). Ainsi tous les atomes de Gd sont directement complexés par des molécules de DOTA, et il reste une partie significative de DOTA non complexés.

La solution est placée dans une membrane de dialyse de 4-6kDa, et est purifiée par dialyse dans un volume d'eau permutée dix fois plus important. Le bain de dialyse est renouvelé deux fois, après 24h et 48h. La dialyse est arrêtée après 72h.

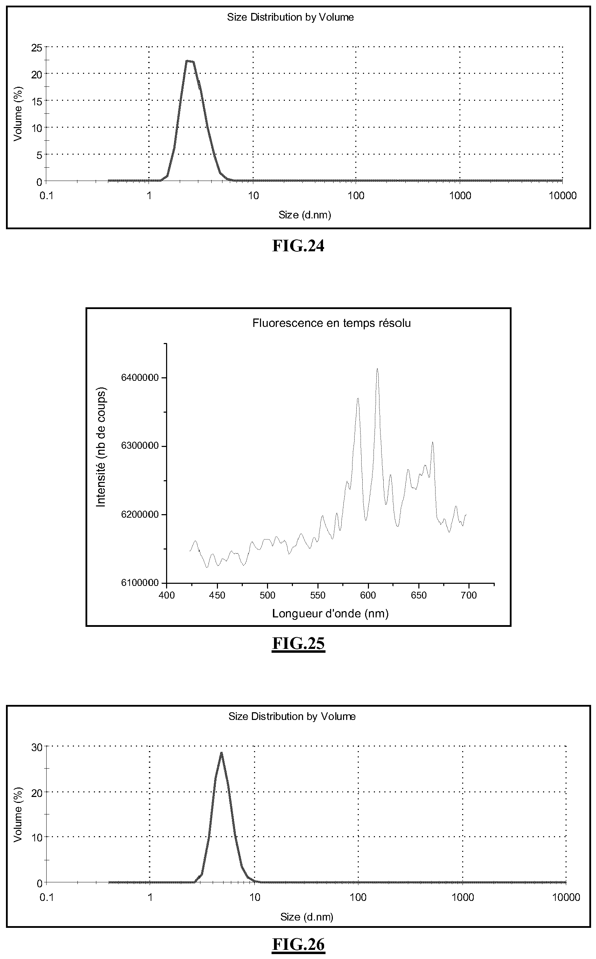

Le contenu des membranes de dialyse est récupéré. La solution apparaît légèrement trouble, elle est donc filtrée à travers un filtre 0,2 µm pour éliminer les impuretés et les particules de grande taille. La taille finale des nanoparticules est d'environ 3 nm. La figure 24 annexée montre la distribution de taille des particules fonctionnalisées DOTA anhydre, mesurée dans l'eau par PCS ; moyenne : 2,7 nm.

D'autre part, lorsque les particules sont centrifugées dans des tubes de Vivaspin, on observe qu'elles ne sont pas retenues par une membrane de 10kDa. Elles sont en revanche retenues par une membrane de 5kDa.

L'analyse chimique réalisée par EDX donne un rapport atomique gadolinium/silicium de 29,3%.

Enfin, les images TEM montrent qu'aucun coeur n'est visible dans ces particules, alors que l'analyse chimique locale prouve la présence de gadolinium dans ces zones.

La solution de nanoparticules peut être conservée plusieurs mois au réfrigérateur après lyophilisation.

### Exemple 12. Dopage du signal magnétique des particules fonctionnalisées DOTA anhydre, sans coeur

Du chlorure de gadolinium est dissous dans de l'eau, et le pH de la solution est ramené à 6. Les ions Gd³⁺ ainsi obtenus sont ajoutés aux particules de l'exemple 11, en excès. On laisse la solution agiter pendant 24h. On purifie la solution par filtration tangentielle pour éliminer les ions Gd³⁺ qui ne se seraient par fixés aux molécules de DOTA.

Après le traitement aux ions Gd³⁺, le signal en relaxométrie r1 des particules a augmenté de 66%. D'autre part, l'analyse chimique réalisée par EDX sur ces particules donne un rapport atomique gadolinium/silicium de 47,5%, soit une augmentation de 62% par rapport aux particules de l'exemple 11. Ces deux mesures concordantes montrent qu'on avait bien des molécules de DOTA libres à la surface des particules, et que ces DOTA ont pu servir à la complexation des ions Gd³⁺ libres..

### Exemple 13. Angiographie

Un lot lyophilisé de nanoparticules de l'exemple 12, contenant 7 µmol de gadolinium, a été redispersé dans 90µL d'eau. Un rat Fisher a été anesthésiés sous isoflurane® (anesthésique gazeux), et la solution de nanoparticules a été injectée dans la veine caudale. Le rat a ensuite subi un examen IRM (séquence t1) pour une étude angiographique. Une augmentation importante du signal a été observée au niveau de l'artère cérébrale moyenne chez cet animal comparé aux animaux contrôle.

### Exemple 14. Dopage à l'europium des particules fonctionnalisées DOTA anhydre, sans coeur

Du chlorure d'europium est dissous dans de l'eau, et le pH de la solution est ramené à 6. Les ions Eu³⁺ ainsi obtenus sont ajoutés aux particules de l'exemple 11, en excès. On laisse la solution agiter pendant 24h. On purifie la solution par filtration tangentielle pour éliminer les ions Eu³⁺ qui ne se seraient par fixés aux molécules de DOTA.

Après le traitement aux ions Eu³⁺, l'analyse chimique réalisée par EDX sur ces particules donne un rapport atomique gadolinium/silicium de 20,1%, et un rapport europium/silicium de 28,8%. Cette mesure montre qu'on avait bien des molécules de DOTA libres à la surface des particules, et qu'elles ont pu être chélatées par ajout d'ions Eu³⁺ libres. D'autre part, le rapport total terres rares/silicium obtenu est de 48,9%, ce qui est cohérent avec le rapport terres rares/silicium des particules de l'exemple 12, qui est de 47,5%. Ceci donne une estimation du nombre total de DOTA à la surface des particules.

Une mesure de fluorescence en temps résolu montre que les ions Eu³⁺ se trouvent principalement à l'état complexé dans la solution. La figure 25 annexée montre ce spectre de fluorescence. L'appareil utilisé pour la fluorescence en temps résolu est un appareil réalisé par la société AXINT, pour les besoins spécifiques du laboratoire.

Des expériences de dopage similaires ont été réalisées avec des ions cuivre et gallium, et ont montré qu'il était possible de complexer ces ions également dans les DOTA libres.

### Exemple 15. Synthèse directe de nanoparticules fonctionnalisées DOTA anhydre, sans coeur, avec saturation des DOTA de surface en gadolinium

Des nanoparticules sont synthétisées selon le protocole décrit dans l'exemple 8, mais la fonctionnalisation de surface se fait avec un large excès de DOTA anhydre (2 DOTA/Gd). Ainsi tous les atomes de Gd sont directement consommés par les complexes de DOTA.

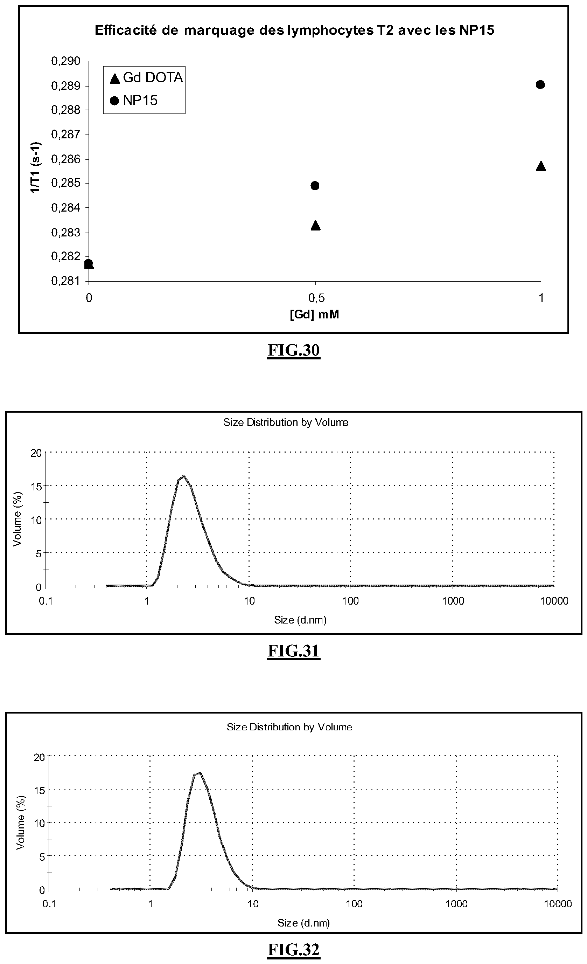

Les nanoparticules sont précipitées dans des bidons de 500 mL d'acétone. Puis l'acétone est retirée, et les particules sont redispersées dans de l'eau. Elles sont ensuite purifiées par filtration tangentielle. Les impuretés de grande taille sont retirées par filtration à l'aide d'une seringue, à travers une membrane de 0,2 µm. La figure 31 annexée montre la distribution de taille des particules fonctionnalisées DOTA anhydre, mesurée dans l'eau par PCS ; moyenne : 2,8 nm.

La solution de nanoparticules peut être conservée plusieurs mois au réfrigérateur après lyophilisation.

### Exemple 16. Marquage cellulaire

Des lymphocytes humains T2 (ATCC n° CRL-1992™) ont été incubés dans l'HBSS (Hank's Balanced Salt Solution) pendant 1h avec les nanoparticules de l'exemple 15 (NP15), avec des concentrations en gadolinium de 0,2 ; 0,5 et 1mM. Après incubation, les cellules ont été lavées 2 fois dans l'HBSS.

La viabilité a été évaluée par numération sur grille de Malassez après ajout de bleu Trypan (dilution au 1/20^{ème}). Pour les trois concentrations en gadolinium présentes dans le milieu d'incubation, la viabilité des cellules était bonne car supérieure à 90%. Dans cette gamme de concentrations, les nanoparticules de l'exemple 15 n'étaient pas toxiques.

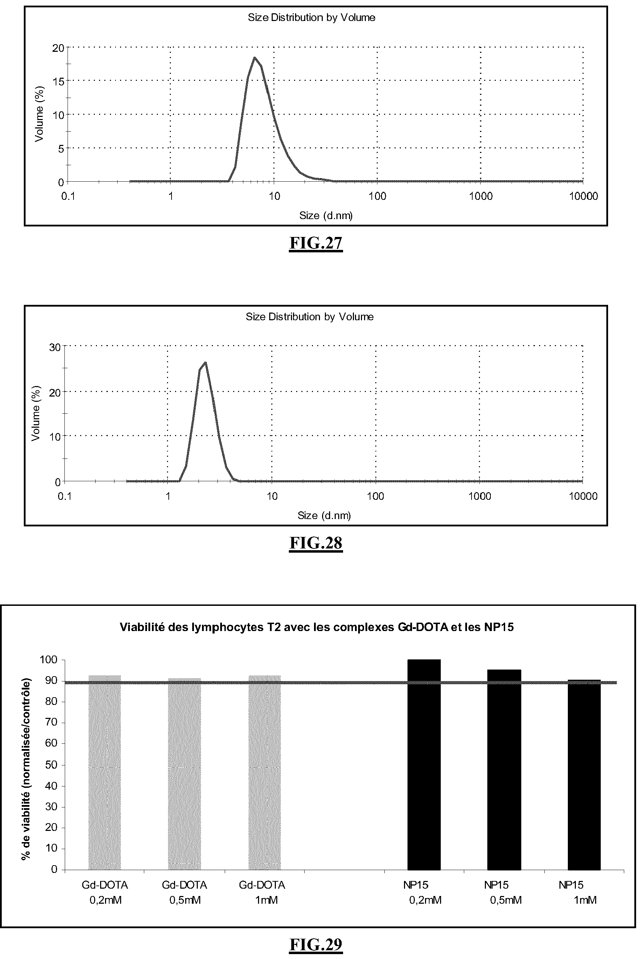

La figure 29 annexée montre le taux de viabilité des lymphocytes T2 pour chaque condition d'incubation.

Après évaluation de la viabilité, les cellules ont été fixées dans une solution de paraformaldéhyde à 4% puis l'efficacité de marquage a été évaluée par mesure du temps de relaxation longitudinal T₁. Plus r₁=1/T₁ était élevée, meilleur était le marquage. Le marquage avec les nanoparticules de l'exemple 15 était donc meilleur qu'avec les complexes Gd-DOTA. A la concentration de 1mM, l'augmentation de signal était de 15% avec 50 000 cellules/mm3. La figure 30 annexée montre l'efficacité de marquage des particules de l'exemple 15 en comparaison avec les complexes Gd-DOTA, et en fonction de la concentration en gadolinium.

D'autre part, la relaxivité des particules de l'exemple 15 a été mesurée en relaxométrie, les quantités de gadolinium réelles étant mesurées par ICP, sur un appareil Varian 710-ES. On obtient une relaxivité r₁ (de l'espèce gadolinium) = 11,4 s⁻¹mM⁻¹ (de gadolinium)⁻¹.s⁻¹ à 50MHz, soit environ trois fois plus élevée que celle du complexe Gd-DOTA seul (ramenée à l'espèce gadolinium), qui est de 3,8 s⁻¹mM⁻¹ (de gadolinium)⁻¹.s⁻¹ à 50MHz.

### Exemple 17. Synthèse de nanoparticules fonctionnalisées DOTA anhydre, sans coeur, en deux étapes successives

Des nanoparticules sont synthétisées selon le protocole décrit dans l'exemple 8, mais la fonctionnalisation de surface se fait avec une quantité de DOTA égale à 0,6 DOTA/Gd.

Une solution de 240 mL d'eau contenant du DTPA en très large excès a été préparée. Le pH de la solution a été ajusté à 6,5. Cette solution a été ajoutée à 160 mL de la solution de nanoparticules. Le mélange a été laissé sous agitation pendant 48h. Ainsi le DTPA libre en très large excès a permis de dissoudre le coeur des particules par complexation forcée.

La solution a ensuite été purifiée par filtration tangentielle. Les impuretés de grande taille ont été retirées par filtration à l'aide d'une seringue, à travers une membrane de 0,2 µm. La figure 32 annexée montre la distribution de taille des particules fonctionnalisées DOTA anhydre, mesurée dans l'eau par PCS ; moyenne : 3,5 nm.

La solution de nanoparticules peut être conservée plusieurs mois au réfrigérateur après lyophilisation.

D'autre part, la relaxivité des particules a été mesurée en relaxométrie, les quantités de gadolinium réelles étant mesurées par ICP. On obtient une relaxivité r1 (de l'espèce gadolinium) = 11,8 s⁻¹mM⁻¹ (de gadolinium)⁻¹.s⁻¹ à 50MHz, soit environ trois fois plus élevée que celle du complexe Gd-DOTA seul (ramenée à l'espèce gadolinium), qui est de 3,8 s⁻¹mM⁻¹ (de gadolinium)⁻¹.s⁻¹ à 50MHz.

### Exemple 18. Synthèse de coeur d'oxyde de lutécium

Une solution est préparée par dissolution d'une quantité de 2,25 g de sel de chlorure de lutécium (LuCl₃, 6H₂O) dans un volume de 245 mL de DEG. A la solution obtenue, un ajout de 245 mL d'une solution de soude à 0,08 mol/L dans du DEG est effectué, à température ambiante, en 10h.

La taille finale des nanoparticules est d'environ 5 nm. La figure 26 annexée montre la distribution de tailles des coeurs d'oxyde de lutécium, mesurée dans le DEG par PCS ; moyenne : 5,0 nm.

### Exemple 19. Fonctionnalisation des coeurs d'oxyde de lutécium avec du DTPABA

Les 490 mL de solution de coeurs de lutécium de l'exemple 18 sont dilués par deux, avec 490 mL de DEG. Autour de ces coeurs, une couche de polysiloxane fonctionnalisé est synthétisée par voie sol-gel. Pour ce faire, la solution de coeurs est portée à 40°C dans un bain d'huile, sous agitation. 811 µL d'APTES, 516 µL de TEOS et 1965 µL d'une solution aqueuse de triéthylamine à 0,1 mol/L sont ajoutés à la solution de coeurs. La solution est laissée ensuite 48h à 40°C sous agitation.

On obtient des particules coeur-écorce d'une taille de 8 nm environ, avec des fonctions amines en surface. La figure 27 annexée montre la distribution de tailles des coeurs d'oxyde de lutécium enrobés polysiloxane ; moyenne : 8,3 nm.

Ensuite 0,1 g de DTPABA est dispersé dans 2 mL de DMSO. Puis 12 mL de la solution de coeurs sont ajoutés à la solution de DTPABA. Le mélange est agité pendant 24h.

Les nanoparticules sont ensuite précipitées dans 100 mL d'acétone. Puis l'acétone est retirée, et les particules sont redispersées dans 20 mL d'eau. Elles sont ensuite purifiées par filtration tangentielle. On obtient des particules d'une taille de 2,5 nm environ. Le DTPABA, en large excès par rapport au lutécium (4 DTPABA/Lu), a dissous le coeur des particules en partie, d'où leur petite taille. La figure 28 annexée montre la distribution de tailles des particules fonctionnalisées DTPA ; moyenne : 2,4 nm.

La solution de nanoparticules peut être conservée plusieurs mois au réfrigérateur après lyophilisation.

### Exemple 20. Complexation des particules d'oxyde de lutécium avec des ions Gd³⁺

Du chlorure de gadolinium est dissous dans de l'eau, et le pH de la solution est ramené à 6. Les ions Gd³⁺ ainsi obtenus sont ajoutés aux particules de lutécium de l'exemple 19, en excès. On laisse la solution agiter 24h. On purifie la solution par filtration tangentielle pour éliminer les ions Gd³⁺ qui ne se seraient pas fixés aux molécules de

### DTPABA.

Les particules initiales de lutécium ne montraient aucun signal en relaxométrie. En revanche, après le traitement aux ions Gd³⁺, un signal de T₁=130 ms apparaît en relaxométrie, après une dilution de la solution par 10. Cette observation confirme la présence de molécules de DTPABA greffées sur les particules, qui ont formé des complexes avec les ions Gd³⁺ ajoutés à la solution.

## Revendications

1. Composition pour son utilisation en thérapie, ou dans l'aide au diagnostic comme agent de contraste, ladite composition comprenant des nanoparticules, lesdites nanoparticules comprenant chacune
• une matrice polyorganosiloxane (POS) incluant des cations Mⁿ⁺ (n=2 à 6) de préférence de terre rare, éventuellement associé à des cations dopants Dm+ (m=2 à 6), de préférence une terre rare différente de M, un actinide et/ou un élément de transition,
• un greffon de fonctionnalisation C¹ chélatant qui est :
* issu d'un chélatant C1,
* lié à la matrice POS par liaison covalente -Si-C-, et,
* en quantité suffisante pour pouvoir complexer tous les cations Mⁿ⁺, et,
• un diamètre d₁ compris entre 1 et 10 nm.

2. Composition pour son utilisation selon la revendication 1, en thérapie comme agent de radiosensibilisation, et **caractérisée en ce que** M est choisi parmi les lanthanides et plus particulièrement Lu, Yb, Gd, et Ho.

3. Composition pour son utilisation selon la revendication 2, dans le traitement du cancer.

4. Composition pour son utilisation selon la revendication 1, dans l'aide au diagnostic comme agent de contraste en imagerie par résonance magnétique, et **caractérisée en ce que** M est choisi parmi Gd et Dy.

5. Composition pour son utilisation selon l'une au moins des revendications précédente, **caractérisée en ce que** la matrice POS de chaque nanoparticule est obtenue à partir d'une nanoparticule précurseur de diamètre d₀ comprenant:
- un coeur à base d'un oxyde métallique M ; et,
- au moins une couche d'enrobage à base de POS ;
ladite nanoparticule précurseur étant soumise à une dissolution de son coeur à base d'un oxyde métallique de sorte que le diamètre de la nanoparticule est réduit à une valeur d₁ comprise entre 1 et 10 nm.

6. Composition pour son utilisation selon l'une au moins des revendications précédentes **caractérisées en ce que** le diamètre d₁ des nanoparticules est compris entre 1 et 5 nm.

7. Composition pour son utilisation selon l'une au moins des revendications précédentes, **caractérisées en ce que** M est un élément gadolinium Gd et le chélatant C1 est choisi parmi les agents chélatants du groupe des acides polycarboxyliques aminés.

8. Composition pour son utilisation selon la revendication 5, **caractérisée en ce que** lesdites nanoparticules précurseurs sont formées par des coeurs d'oxyde de gadolinium enrobés de polysiloxanes et fonctionnalisés par un complexant DOTAGA anhydride de formule suivante :

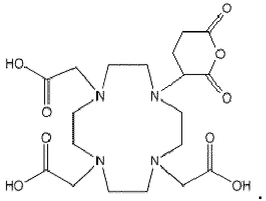

9. Composition pour son utilisation selon la revendication 8, **caractérisées en ce que** son diamètre d₁ est compris entre 3 et 5 nm.

10. Composition pour son utilisation selon l'une au moins des revendications précédentes **caractérisées par** leur masse molaire (en kDa) supérieure ou égale à 5 kDa et inférieure ou égale à 20 kDa.

11. Composition pour son utilisation selon l'une au moins des revendications précédentes **caractérisée par** un rapport de % atomique [(M/Si) x100] compris entre 10 et 60.

12. Composition pour son utilisation selon l'une au moins des revendications précédentes **caractérisées en ce que** la relaxivité r₁ par ion Mⁿ⁺ est supérieure à 5 mM⁻¹(d'ion Mⁿ⁺).s⁻¹ préférentiellement 10 mM⁻¹(d'ion Mⁿ⁺).s⁻¹ pour une fréquence de 20 MHz.

13. Composition pour son utilisation selon l'une au moins des revendications précédentes **caractérisées en ce que** la relaxivité r₁ par ion Mⁿ⁺ à 60 MHz est supérieure à la relaxivité r₁ par ion Mⁿ⁺ à 20 MHz.

14. Composition pour son utilisation selon l'une au moins des revendications précédentes, **caractérisée en ce que** les nanoparticules ne comprennent pas de coeur cristallisé.

15. Composition pour son utilisation selon l'une au moins des revendications précédentes, **caractérisée** en qu'elle est constituée :
**(i)** d'une matière solide obtenue par lyophilisation d'une suspension liquide comprenant lesdites nanoparticules, ou
**(ii)** d'un liquide injectable comprenant lesdites nanoparticules.
